(19) Europäisches Patentamt
European Patent Office
Office européen des brevets

(11) EP 3 247 767 B1

(12) **EUROPÄISCHE PATENTSCHRIFT**

(45) Veröffentlichungstag und Bekanntmachung des Hinweises auf die Patenterteilung:
**26.12.2018 Patentblatt 2018/52**

(21) Anmeldenummer: **16702347.2**

(22) Anmeldetag: **20.01.2016**

(51) Int Cl.:
*C09K 11/06* (2006.01) *C07D 209/00* (2006.01)
*H01L 51/50* (2006.01) *C07D 401/10* (2006.01)
*C07D 413/12* (2006.01) *C07D 417/10* (2006.01)
*H01L 51/00* (2006.01) *C07D 405/10* (2006.01)
*C07D 409/10* (2006.01)

(86) Internationale Anmeldenummer:
**PCT/EP2016/051143**

(87) Internationale Veröffentlichungsnummer:
**WO 2016/116508 (28.07.2016 Gazette 2016/30)**

(54) **ORGANISCHE MOLEKÜLE, INSBESONDERE ZUR VERWENDUNG IN OPTOELEKTRONISCHEN BAUELEMENTEN**

ORGANIC MOLECULES, IN PARTICULAR FOR USE IN OPTOELECTRONIC COMPONENTS

MOLÉCULES ORGANIQUES À UTILISER EN PARTICULIER DANS DES COMPOSANTS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2015 EP 15151870**
**20.05.2015 EP 15168399**

(43) Veröffentlichungstag der Anmeldung:
**29.11.2017 Patentblatt 2017/48**

(73) Patentinhaber: **cynora GmbH**
**76646 Bruchsal (DE)**

(72) Erfinder:
- **DANZ, Michael**
  **76344 Eggenstein-Leopoldshafen (DE)**
- **FLÜGGE, Harald**
  **76135 Karlsruhe (DE)**
- **FRIEDRICHS, Jana**
  **76137 Karlsruhe (DE)**
- **GRAB, Tobias**
  **76133 Karlsruhe (DE)**
- **JACOB, Andreas**
  **30449 Hannover (DE)**
- **SEIFERMANN, Stefan**
  **77815 Bühl (DE)**
- **VOLZ, Daniel**
  **76137 Karlsruhe (DE)**
- **AMBROSEK, David**
  **10437 Berlin (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**EP-A1- 2 141 152** **EP-A1- 2 461 390**
**WO-A1-2014/146750** **JP-A- 2004 253 298**
**JP-A- 2009 021 336**

- **KAMIKAWA T ET AL: "PALLADIUM CATALYSTS FOR CROSS-COUPLING OF ORTHO-SUBSTITUTED ARYL TRIFLATES WITH GRIGNARD REAGENTS", SYNLETT, GEORG THIEME VERLAG, DE, 1. Februar 1997 (1997-02-01), Seite 163/164, XP001121906, ISSN: 0936-5214, DOI: 10.1055/S-1997-750**
- **KAORU FUJI ET AL: "Unexpectedly facile racemization of 8-diphenylphosphinoyl-8?-methoxy-1,1?-bina phthyl", CHEMICAL COMMUNICATIONS - CHEMCOM, Nr. 14, 1. Januar 1996 (1996-01-01), Seite 1609, XP055222961, GB ISSN: 1359-7345, DOI: 10.1039/cc9960001609**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 247 767 B1

• TAMOTSU SUGIMORI ET AL: "Phthalocyanines Obtained from Phthalonitriles with Phenyl Derivatives. A New Method for the Synthesis of the Phthalonitriles by Use of Suzuki-Coupling Reaction.", CHEMISTRY LETTERS, Nr. 10, 1. Januar 2000 (2000-01-01), Seiten 1200-1201, XP055262306, JAPAN ISSN: 0366-7022, DOI: 10.1246/cl.2000.1200

## Beschreibung

Die Erfindung betrifft rein organische Moleküle und deren Verwendung in organischen lichtemittierenden Dioden (OLEDs) und in anderen optoelektronischen Bauelementen.

## Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(I)-Komplexe verwendet werden, die aufgrund eines geringen Energieabstandes zwischen dem untersten Triplett-Zustand $T_1$ und dem darüberliegenden Singulett-Zustand $S_1$ ($\Delta E(S_1\text{-}T_1)$ Triplett-Exitonen thermisch in einen Singulett-Zustand rückbesetzen können. Neben der Verwendung von Übergangsmetallkomplexen können auch rein organische Moleküle diesen Effekt ausnutzen.

Einige solcher TADF-Materialien wurden bereits in ersten optoelektronischen Bauelementen eingesetzt. Die bisherigen Lösungen weisen jedoch Nachteile und Probleme auf: Die TADF-Materialien weisen in den optoelektronischen Bauelementen oftmals keine ausreichende Langzeitstabilität, keine ausreichende thermische oder keine ausreichende chemische Stabilität gegenüber Wasser und Sauerstoff auf. Außerdem sind nicht alle wichtigen Emissionsfarben verfügbar. Weiterhin sind einige TADF-Materialien nicht verdampfbar und dadurch für den Einsatz in kommerziellen optoelektronischen Bauteilen nicht geeignet. Auch weisen einige TADF-Materialien keine passenden Energielagen zu den weiteren im optoelektronischen Bauteil verwendeten Materialien (z.B. HOMO-Energien von TADF-Emittern von größer gleich - 5,9 eV) auf. Nicht mit allen TADF-Materialien lassen sich ausreichend hohe Effizienzen der optoelektronischen Bauelemente bei hohen Stromdichten bzw. hohe Leuchtdichten erreichen. Weiterhin sind die Synthesen einiger TADF-Materialien aufwendig.

Weiter sind aus Sugimori et al. (Chemistry Letters 2000, 10, 1200-1201) Phytalocyanine mit peripheren Phenylgruppen bekannt, die durch Suzuki-Miyaura Kupplung hergestellt werden können.

Aus der EP 2 141 152 A1 sind organische elektrolumineszierende Bauelemente bekannt.

Auch aus der EP 2 461 390 A1 sind organische elektrolumineszierende Bauelemente bekannt.

## Beschreibung

Die Erfindung betrifft in einem ersten Aspekt die Bereitstellung von organischen Molekülen, die eine Struktur der Formel 1 aufweisen oder eine Struktur der Formel 1 haben:

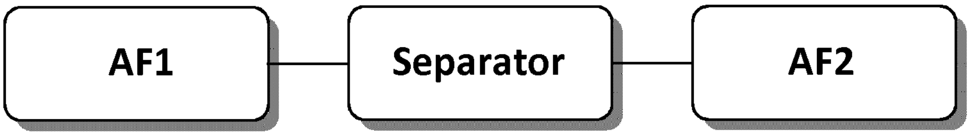


**Formel 1**

wobei gilt:

AF1: eine erste chemische Einheit, aufweisend ein konjugiertes System, insbesondere mindestens sechs in Konjugation stehende $\pi$-Elektronen (z. B. in Form mindestens eines aromatischen Systems);
AF2: eine zweite chemische Einheit, aufweisend ein konjugiertes System, insbesondere mindestens sechs in Konjugation stehende $\pi$-Elektronen (z. B. in Form mindestens eines aromatischen Systems);
AF1 $\neq$ AF2;
Separator ist eine chemische Einheit, die AF1 und AF2 verknüpft;
wobei der Separator S eine Struktur der Formel 2 aufweist

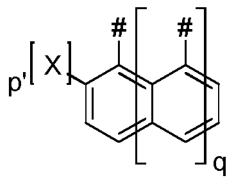

**Formel 2**

wobei die Anknüpfungspositionen für die chemischen Einheiten AF1 und AF2 durch den Platzhalter # gekennzeichnet sind,
X = #, $CH_2$#, oder

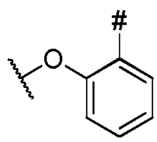

p' = 0 oder 1;
q = 0 oder 1;
wobei p' $\neq$ q;
wobei insbesondere

- die Differenz der Energie des HOMO der zweiten chemischen Einheit AF2 und der Energie des HOMO der ersten chemischen Einheit AF1 > 0,8 eV ist ($\Delta$ HOMO = HOMO(AF2) - HOMO(AF1) > 0,8 eV);
- die Differenz der Energie des LUMO der zweiten chemischen Einheit AF2 und der Energie des LUMO der ersten chemischen Einheit AF1 > 0,8 eV ist ($\Delta$ LUMO = LUMO(AF2) - LUMO(AF1) > 0,8 eV) und
- die Differenz der Energie des LUMO der ersten chemischen Einheit AF1 und der Energie des HOMO der zweiten chemischen Einheit AF2 > 0,9 eV ist ($\Delta$ Gap = LUMO (AF1) - HOMO(AF2)> 0,9 eV);

und wobei insbesondere die Energie des HOMO des Separators S kleiner ist als die Energie des HOMO der zweiten chemischen Einheit AF2 (HOMO(Separator) < HOMO(AF2));
und wobei insbesondere die Energie des LUMO des Separators S des organischen Moleküls größer ist als die Energie des LUMO der ersten chemischen Einheit AF1 des organischen Moleküls (LUMO(Separator) > LUMO(AF1)).

In einer Ausführungsform der Erfindung sind die chemischen Einheiten AF1 und AF2 derart miteinander verknüpft, dass die elektronische Kommunikation zwischen ihnen unterbrochen wird. Diese Unterbrechung ist durch eine Lokalisierung der Grenzorbitale HOMO und LUMO auf separaten Molekülteilen gekennzeichnet, sodass ein Charge-Transfer Übergang ermöglicht wird.

In einer Ausführungsform der Erfindung sind die chemischen Einheiten AF1 und AF2 derart über einen Separator miteinander verbunden, dass die elektronische Kommunikation zwischen ihnen unterbrochen wird, was anhand der Lokalisierung der Grenzorbitale HOMO und LUMO auf separaten Molekülteilen gekennzeichnet ist. Der Separator kann dabei eine beliebige Struktur aufweisen, solange eine Unterbrechung der elektronischen Kommunikation gewährleistet wird.

Von Vorteil ist, wenn der Separator die elektronische Kommunikation von AF1 und AF2 über konjugierte Bindungen miteinander unterbricht. Unterbrechung der elektronischen Kommunikation über konjugierte Bindungen bedeutet dabei, dass die Orbitale HOMO und LUMO weit genug voneinander entfernt sind, sodass keine signifikante Überlappung stattfinden kann, wobei ein maximaler Überlapp der Orbitale von 20% des Gesamtvolumens der Orbitale erlaubt ist. Wird dieser Wert überschritten, so wird von einer signifikanten Überlappung gesprochen. Eine Bestimmung und Quantifizierung der Unterbrechung bzw. des Überlapps kann beispielsweise durch quantenchemische Standardverfahren wie DFT-Rechnungen erfolgen, in dem der Orbitalüberlapp der Wellenfunktionen HOMO und LUMO bestimmt wird. Die elektronische Kommunikation zwischen den zwei chemischen Einheiten AF1 und AF2 über konjugierte Bindungen mit einem optionalen Separator ist unterbrochen, wenn die Grenzorbitale HOMO und LUMO auf separaten Molekülteilen lokalisiert sind, sodass ein Charge-Transfer Übergang ermöglicht wird. Die Lokalisierung der Grenzorbitale HOMO oder LUMO wird dabei mithilfe der Dichtefunktionaltheorie (DFT) mit dem BP86-Funktional (Becke, A. D. Phys. Rev. A1988, 38, 3098-3100; Perdew, J. P. Phys. Rev. B1986, 33, 8822-8827) visualisiert: Aus der Einelektronen-Wellenfunktion wird die Einelektronendichte durch Quadrieren berechnet und über den Raum integriert, den der untersuchte Molekülteil einnimmt. Dieser Raum kann aus den Atomkoordinaten und den van-der-Waals-Radien der Atome bestimmt werden. Die resultierende Zahl entspricht dem Anteil des Orbitals auf dem Molekülteil. Eine mehrheitliche Trennung der Grenzorbitale entspricht dabei einem Überlappungsparameter O im Bereich 0.1-20%, um einen Charge-Transfer Übergang zu ermöglichen. Der Überlappungsparameter O zwischen der HOMO-Wellenfunktion $\phi_a$ und der der LUMO-Wellenfunktion $\phi_b$ ergibt sich aus dem Integral über den gesamten Raum über den jeweils kleineren Wert der quadrierten Wellenfunktion:

$$O = \int \rho \, \mathrm{d}\tau \text{ mit } \rho = \begin{cases} \varphi_a^2, & \text{wenn } |\varphi_a| < |\varphi_b| \\ \varphi_b^2, & \text{wenn } |\varphi_a| \geq |\varphi_b| \end{cases}$$

Dabei werden die Energiewerte HOMO(AF1), HOMO(AF2), LUMO(AF1), LUMO(AF2) mithilfe der Dichtefunktionaltheorie (DFT) berechnet, wobei die Anknüpfungspositionen der ambifunktionalen Einheiten und der Separatoren mit einem Wasserstoffatom entsprechend ihrer chemischen Valenzen abgesättigt werden. Die angegebenen Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827).

In einer Ausführungsform der Erfindung ist der Separator ausgewählt aus der Gruppe der folgenden Strukturen

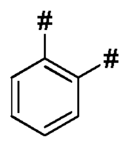

S-a

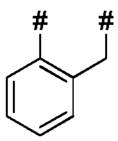

S-b

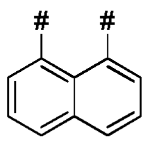

S-c

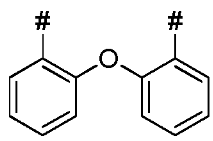

S-e

wobei # die Positionen kennzeichnet, an denen die chemischen Einheiten AF1 und AF2 an den Separator gebunden sind.

In einer erfindungsgemäßen Ausführungsform des organischen Moleküls wird ein Separator S-a bis S-e verwendet, der durch Anknüpfung an AF1/AF2 zu maximal 30 % des LUMO der ersten chemischen Einheit AF1 bzw. zu maximal 30% des HOMOs der zweiten chemischen Einheit AF2 auf dem Separator S des organischen Moleküls lokalisiert.

In einer weiteren erfindungsgemäßen Ausführungsform des organischen Moleküls wird ein Separator S verwendet, der infolge von konjugativer Anknüpfung Teil des LUMOs der AF1 oder Teil des HOMOs der AF2 wird, dabei aber die Energielevels $E_{LUMO}$/$E_{HOMO}$ um maximal 0,4 eV, insbesondere um maximal 0,3 eV verschiebt (siehe Beispiele).

In einer Ausführungsform der Erfindung ist die chemische Einheit AF1 ausgewählt aus der Gruppe der folgenden Strukturen:

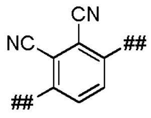

153

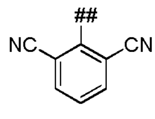

150

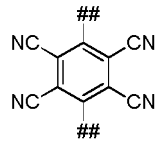

150-1

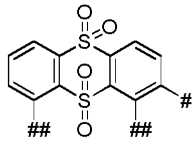

46

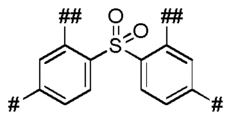

111

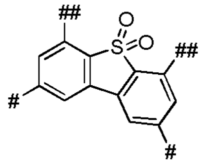

198

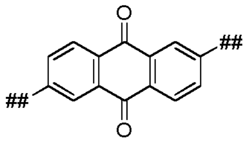

36

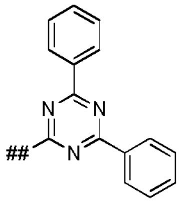

233

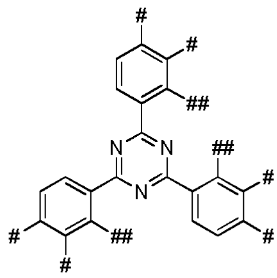

233-1

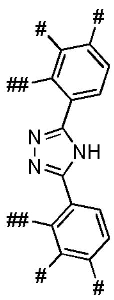

222

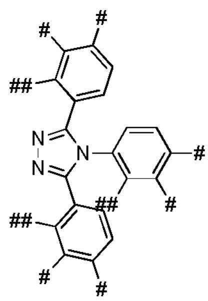

222-1

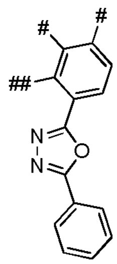

118

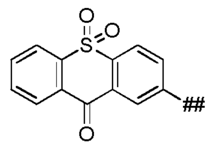

43

wobei gilt

#, ##: Anknüpfungspositionen an den Separator. Die Anknüpfungspositionen # und ## stehen hierbei in ortho-Stellung zu einem Alkyl- oder Arylrest oder die Anknüpfung erfolgt über ein N-Atom. Optional können bei zwei benachbart eingezeichneten Positionen # und/oder ## auch eine der Positionen mit einem Rest R* substiutiert sein, um diese Anforderung zu erfüllen.

Moleküle mit vergleichbarer Grundstruktur wurden hierbei mit der gleichen ersten Zahl benannt und einer weiteren Zahl nummeriert (z. B. 150 und 150-1). Die entsprechend vergleichbar nummerierten organischen Moleküle weisen hierbei identische Werte für ihre jeweiligen HOMO- und LUMO-Energien auf.

In einer Ausführungsform ist die die Akzeptoreinheit AF1 ausgewählt aus 150, 150-1, 46, 233-1, 222, 222-1 und 118, in denen die Anknüpfungsposition ## ein sterisch anspruchsvolles, aromatisches C-Atom ist. Hierdurch wird gewährleistet, dass eine Verdrillung der Reste erreicht wird.

Ein sterisch anspruchsvolles, aromatisches C-Atom bedeutet dabei, dass in mindestens einer ortho-Position zu diesem C-Atom ein Substituent ungleich H vorhanden ist.

In einer Ausführungsform der Erfindung ist die chemische Einheit AF2 ausgewählt aus der Gruppe der folgenden Strukturen:

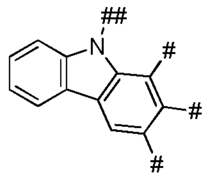
328

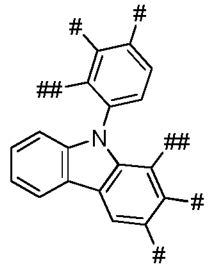
55

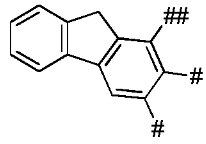
106

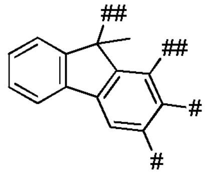
106-1

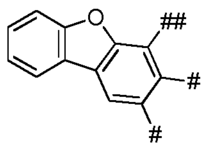
432

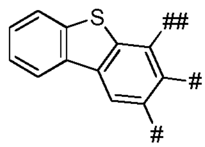
433

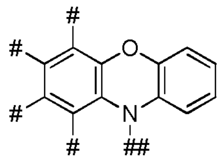
41

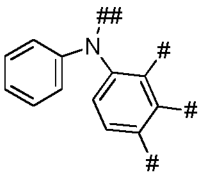
223

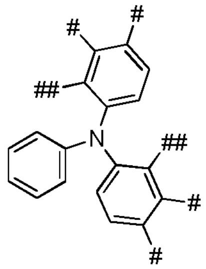
317

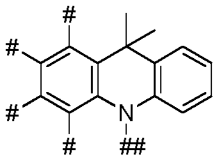
337

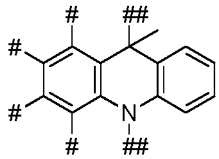
337-1

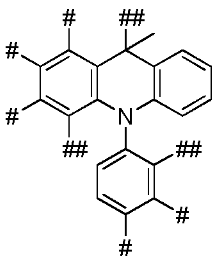
337-2

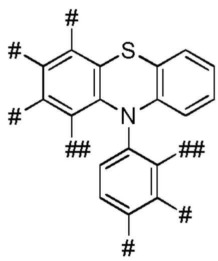
77

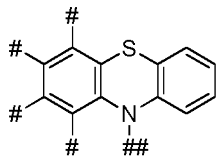
77-1

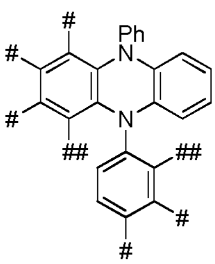
425

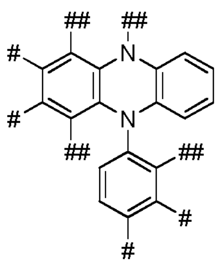
425-1

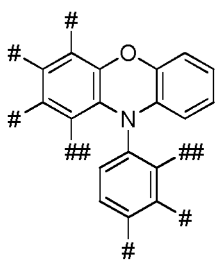
78

und wobei gilt:

#, ##: Anknüpfungspositionen an den Separator. Die Anknüpfungspositionen # und ## stehen hierbei in ortho-Stellung zu einem Alkyl- oder Arylrest oder die Anknüpfung erfolgt über ein N-Atom. Optional können bei zwei benachbart eingezeichneten Positionen # und/oder ## auch eine der Positionen mit einem Rest R* substituiert sein,

um diese Anforderung zu erfüllen.

Die Anknüpfung der chemischen Einheit AF2 erfolgt entsprechend über ein Kohlenstoffatom oder über ein Stickstoffatom. Bei den Anknüpfungspositionen ## handelt es sich um sterisch anspruchsvolle Kohlenstoff- oder Stickstoffatome. Hierdurch wird gewährleistet, dass eine Verdrillung der Reste relativ zueinander erreicht wird.

Moleküle mit vergleichbarer Grundstruktur wurden hierbei mit der gleichen ersten Zahl benannt und einer weiteren Zahl nummeriert (z. B. 106 und 106-1). Die entsprechend vergleichbar nummerierten organischen Moleküle weisen hierbei nahezu identische Werte für ihre jeweiligen HOMO- und LUMO-Energien auf.

Optional können in nicht erfindungsgemäßen Molekülen alle in AF1, AF2 und im Separator vorhandenen C-H Einheiten durch C-R* ersetzt sein,

mit

R* ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden; in einer Ausführungsform können zwei oder mehrere dieser Substituenten R* auch miteinander ein mono- oder polycyclisches, aliphatisches, aromatisches und/oder benzoannelliertes m Ringsystem bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$,$-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert

sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$,

-C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-,$-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind insbesondere N, O, und S. Werden in der Beschreibung der vorliegenden Erfindung andere, hiervon abweichende Angaben gemacht, beispielsweise bezüglich der Zahl der aromatischen Ringatome oder der enthaltenen Heteroatome, so gelten diese.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind insbesondere ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (insbesondere weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch System wie 9,9'-Diarylfluoren, Triarylamin, Diarylether, Stilben etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppen oder durch eine Silylgruppe verbunden sind. Weiterhin werden auch Systeme, in denen zwei oder mehr Aryl- oder Heteroyraylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen.

In einer speziellen Ausführungsform der Erfindung weisen die organischen Moleküle eine Struktur der Formel 3 auf,

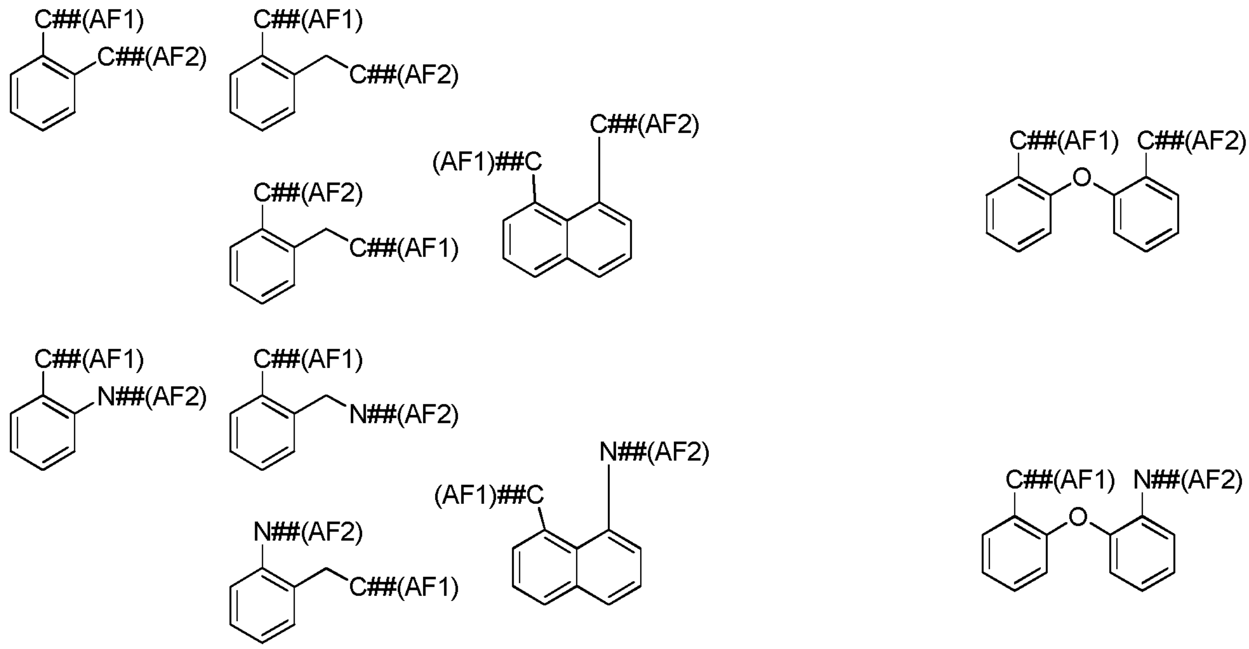

**Formel 3**

wobei die Anknüpfung der chemischen Einheit AF1 an den Separator über ein sterisch anspruchsvolles aromatisches C-Atom ## der chemischen Einheit AF1 erfolgt;
wobei die chemische Einheit AF2 optional über ein sterisch anspruchsvolles C-Atom ## oder über ein Stickstoffatom N## angebunden ist.

In Formel 3 steht C##(AF1) für die chemische Einheit AF1, wobei die Verknüpfung an den Separator über ein sterisch anspruchsvolles aromatisches C-Atom der chemischen Einheit AF1 erfolgt. C##(AF2) steht für die chemische Einheit AF2, wobei die Verknüpfung an den Separator über ein sterisch anspruchsvolles C-Atom der chemischen Einheit AF2 erfolgt. N##(AF2) für die chemische Einheit AF2, wobei die Verknüpfung an den Separator über ein Stickstoffatom der chemischen Einheit AF2 erfolgt.

Erfolgt die Anknüpfung der chemischen Einheit AF2 über ein Stickstoffatom, so wird AF2 ausgewählt aus der Gruppe bestehend aus 328, 41, 223, 77-1, 337-1 und 425-1.

Insbesondere der Separator S unterscheidet die organischen Moleküle funktionell von Molekülen gemäß dem Stand der Technik, da die hier gezeigte Art der Trennung von AFs (oder Donoren und Akzeptoren) bisher noch nicht bekannt ist. Bekannte organische Emitter bestehen in der Regel aus direkt verknüpften chemischen Einheiten. Eine Trennung der konjugierten aromatischen Systeme fand bisher nicht statt, insbesondere in Verbindung mit der Lokalisierung von HOMO und LUMO auf separaten Molekülteilen. Separatoren dienen der Unterbrechung der elektronischen Kommunikation zwischen den chemischen Einheiten AF1 und AF2 durch eine derartige Verknüpfung der Einheiten, dass die Grenzorbitale HOMO und LUMO auf mehrheitlich separaten Molekülteilen liegen, was ohne den Separator nicht zwingend der Fall sein muss.

Separatoren im Sinne dieser Erfindung verändern die Lage des HOMO bzw. LUMO der AFs nicht signifikant. Als nicht signifikant gilt im Rahmen dieser Erfindung eine Veränderung von nicht mehr als +/- 0,4 eV. Die Berechnung derartiger Energien ist bekannt und funktioniert nach der oben beschriebenen Weise per DFT-Rechnung.

Anhand spektroskopischer Auswahlregeln (symmetrische Moleküle) oder durch Messung des Extinktionskoeffizienten (UV/VIS-Spektroskopie) oder quantenchemische Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 50 µs. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.

Ein Maß für die Abklingdauer ist das quantenmechanischen Überlappungsintegral, welches näherungsweise durch den oben definierten Überlappungsparameter O dargestellt wird. Je kleiner das Überlappungsintegral, desto stärker sind die Grenzorbitale HOMO und LUMO getrennt, und umso wahrscheinlicher der Charge-Transfer-Übergang. Jedoch sinkt gleichermaßen die Wahrscheinlichkeit einer TADF-Emission aufgrund abnehmender Oszillatorstärken.

Bei einem Wert von 1 für den Überlappungsparameter O liegt nicht mehr verzögerte Fluoreszenz (TADF) aufgrund eines Charge-Transfer Übergangs vor.

Um eine effiziente TADF-Emission mit kurzen Abklingdauern zu erreichen, muss das Überlappungsintegral gezielt gesteuert werden. Die gewünschte Überlappung wird durch die geeignete Wahl eines erfindungsgemäßen Separators S erreicht.

Ein Maß für die Abklingdauer ist der $\Delta E(S_1\text{-}T_1)$-Abstand. Dieser wird durch die Überlappung von HOMO und LUMO beeinflusst. Die Größe des quantenmechanischen Überlappungsintegrals, welche nach oben genannter DFT-Methode berechenbar ist, kann durch Wahl des Separators gezielt gesteuert werden. Kommt es zur völligen Trennung von HOMO und LUMO hat dieses einen Wert von 0. Die Wahrscheinlichkeit einer effizienten Emission des organischen Moleküls sinkt drastisch. Bei einem Wert von 1 liegt nicht mehr verzögerte Fluoreszenz (TADF) sondern spontane Emission vor. Die gewünschte Überlappung wird durch die geeignete Wahl eines erfindungsgemäßen Separators S erreicht.

Die gezeigten Separatoren S erfüllen dabei insbesondere zwei wesentliche funktionelle Merkmale (s. Figur 2):

- die HOMO-Energie ist niedriger als die HOMO-Energie der als Donor fungierenden chemischen Einheit AF und
- die LUMO-Energie ist höher als die LUMO-Energie der als Akzeptor fungierenden chemischen Einheit AF.

Durch Kombination der oben definierten chemischen Einheiten AF1 und AF2 und der Festlegung der Verknüpfung durch einen Separator ergeben sich die erfindungsgemäßen Moleküle.

In einer Ausführungsform der Beschreibung weist die Einheit AF1 eine Struktur der Formel 1b auf oder hat eine Struktur der Formel 1b

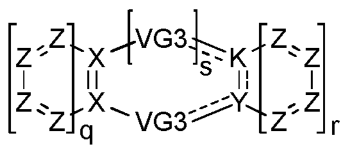

**Formel 1b**

wobei gilt:

q ist 0, 1; r ist 0, 1; s ist 0, 1;

VG3 = verbrückende Gruppe ist bei jedem Auftreten unabhängig voneinander N, O, S, CR**, C, eine Element-Element-Einfachbindung zwischen X und Y oder zwischen X und K, wobei nicht 2 Einheiten VG3 gleichzeitig eine Element-Element-Einfachbindung zwischen X und Y und zwischen X und K sind; BR**, NR**, $GeR^{**}_2$, $AsR^{**}_2$, $SiR^{**}_2$, wobei nicht zwei Einheiten VG3 gleichzeitig gleich BR**, NR**, $GeR^{**}_2$, $AsR^{**}_2$, $SiR^{**}_2$ sind; außerdem:

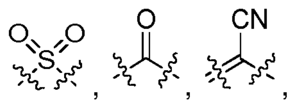,

X ist bei jedem Auftreten unabhängig voneinander C; oder ist CR**, N wenn q = 0;

Y ist C; oder ist CR**, $CR^{**}_2$, N, NR**, O, S wenn r = 0;

K ist Y; oder ist R* wenn r = 0 und gleichzeitig s = 0;

Z ist bei jedem Auftreten unabhängig voneinander CR**, N;

und wobei maximal 4 der Einheiten VG3, K, X, Y, Z gleichzeitig gleich N sind;

und wobei mindestens 1 der Einheiten VG3, K, X, Y, Z gleich einer von C-H verschiedenen Gruppe ist, die mindestens 1 Stickstoff- oder ein Sauerstoff- oder ein Schwefelatom enthält;

R** ist bei jedem Auftreten unabhängig voneinander entweder ein Rest R*, oder kennzeichnet eine chemische Bindung an einen Separator S, wobei genau ein R** eine chemische Bindung an einen Separator S kennzeichnet.

R* ist wie oben definiert.

In einer weiteren Ausführungsform der Beschreibung weist die Einheit AF1 eine Struktur der Unterformeln 1.1 bis 1.23 auf oder hat eine Struktur der Unterformeln 1.1 bis 1.23

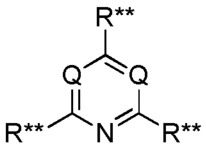
**Unterformel 1.1**

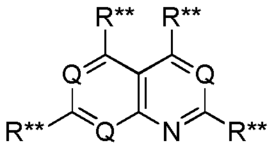
**Unterformel 1.2**

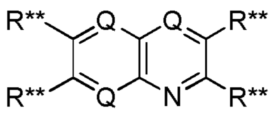
**Unterformel 1.3**

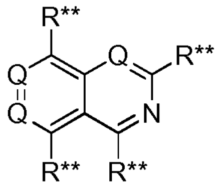
**Unterformel 1.4**

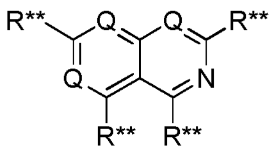
**Unterformel 1.5**

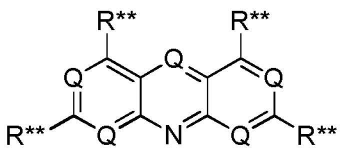
**Unterformel 1.6**

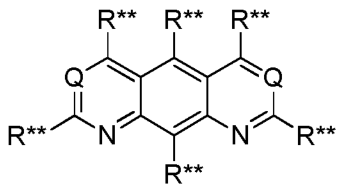
**Unterformel 1.7**

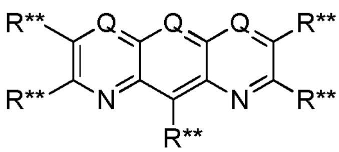
**Unterformel 1.8**

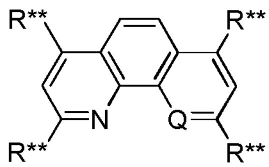
**Unterformel 1.9**

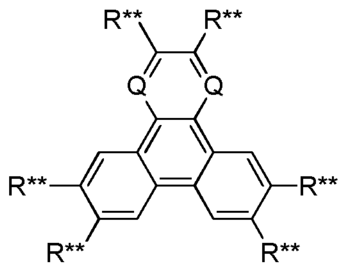
**Unterformel 1.10**

und wobei gilt:

Q ist N, CR**, wobei nicht zwei direkt benachbarte Einheiten Q gleichzeitig gleich N sind;

R** ist bei jedem Auftreten unabhängig voneinander entweder ein Rest R*, oder kennzeichnet eine chemische Bindung an einen Separator S, wobei genau ein R** eine chemische Bindung an einen Separator S kennzeichnet.

R* ist wie oben definiert.

In einer weiteren Ausführungsform der Beschreibung weist die Einheit AF1 eine Struktur der Unterformeln 1.11 bis 1.13 auf oder hat eine Struktur der Unterformeln 1.11 bis 1.13

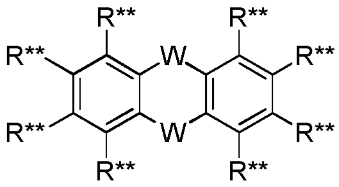

**Unterformel 1.11**

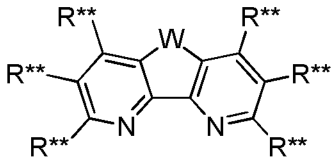

**Unterformel 1.12**

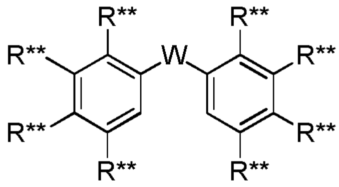

**Unterformel 1.13**

und wobei gilt:

W ist

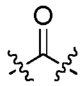, 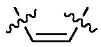, 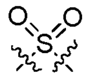,

eine Element-Element-Einfachbindung, wobei nicht zwei Einheiten W gleichzeitig eine Element-Element-Einfachbindung sind, NR**, wobei nicht zwei Einheiten W gleichzeitig gleich NR** sind;

R** ist bei jedem Auftreten unabhängig voneinander entweder ein Rest R*, oder kennzeichnet eine chemische Bindung an einen Separator S, wobei genau ein R** eine chemische Bindung an einen Separator S kennzeichnet.

R* ist wie oben definiert.

In einer weiteren Ausführungsform der Beschreibung weist die Einheit AF1 eine Struktur der Unterformeln 1.14 bis 1.21 auf oder hat eine Struktur der Unterformeln 1.14 bis 1.21

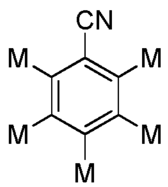

**Unterformel 1.14**

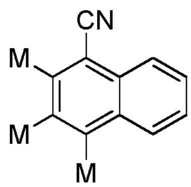

**Unterformel 1.15**

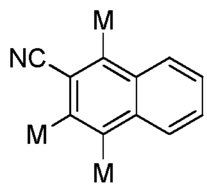

**Unterformel 1.16**

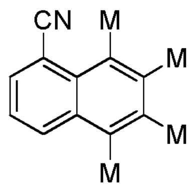

**Unterformel 1.17**

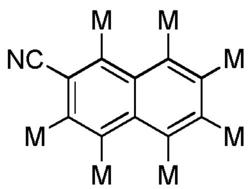

**Unterformel 1.18**

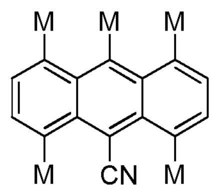

**Unterformel 1.19**

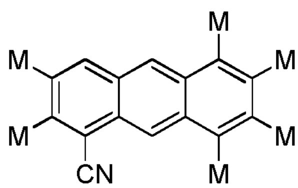

**Unterformel 1.20**

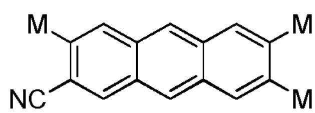

**Unterformel 1.21**

und wobei gilt:

M ist H, Deuterium, Alk, Phenyl, Pyridyl, CN, wobei maximal 4 Einheiten M gleichzeitig gleich CN sind, oder kennzeichnet eine chemische Bindung an den Separator S, wobei genau ein M eine chemische Bindung an einen Separator S kennzeichnet;

Alk ist Methyl, Ethyl, Propyl *iso*-Propyl, Butyl, *tert*-Butyl, Pentyl, Hexyl, 2-Ethylhexyl, Cyclohexyl, Heptyl Octyl, Nonyl, Decyl, Undecyl oder Dodecyl.

In einer weiteren Ausführungsform der Beschreibung weist die Einheit AF1 eine Struktur der Unterformeln 1.22 bis 1.23 auf oder hat eine Struktur der Unterformeln 1.22 bis 1.23

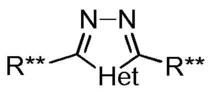 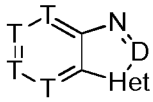

**Unterformel 1.22** **Unterformel 1.23**

und wobei gilt: Het ist NR**, O, S, $SO_2$;
D ist N, CR**;
T ist N, CR**, wobei maximal 2 Einheiten T gleich N sind; und wobei keine benachbarten Einheiten T gleichzeitig gleich N sind;
R** ist bei jedem Auftreten unabhängig voneinander entweder ein Rest R*, oder kennzeichnet eine chemische Bindung an einen Separator S, wobei genau ein R** eine chemische Bindung an einen Separator S kennzeichnet.
R* ist wie oben definiert.

In einer Ausführungsform der Beschreibung weist die Einheit AF2 eine Struktur der Formel 4 auf oder hat eine Struktur der Formel 4

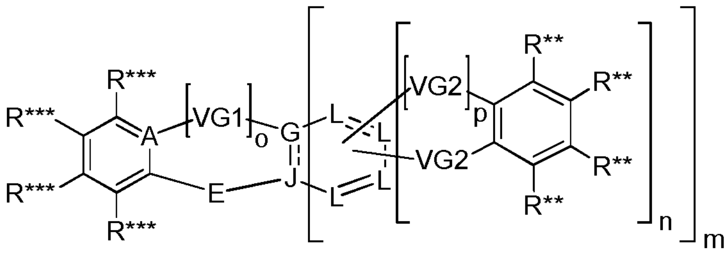

**Formel 4**

wobei gilt:

m ist 0 oder 1;

n ist 0 oder 1, wobei bei m = 0 immer auch n = 0 ist;

o ist 0 oder 1;

p ist 0 oder 1;

A ist CR*** wenn o = 0, ansonsten C;

VG1 = verbrückende Gruppe, ist ausgewählt aus der Gruppe bestehend aus

- NR**, $CR^{**}_2$, O, S und einer C-C-Einfachbindung; oder
- NR**, $CR^{**}_2$, O, S, einer C-C-Einfachbindung, BR**, AsR**, $SiR^{**}_2$, $GeR^{**}_2$,

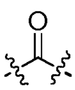, 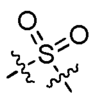, 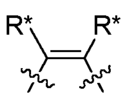, 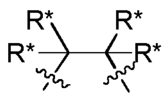, 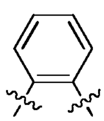, 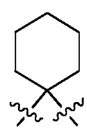, 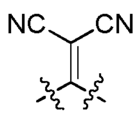, 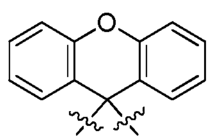,

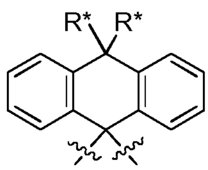, 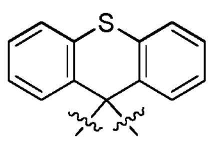, und 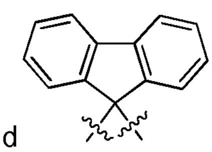,

wenn m = 1 und gleichzeitig n = 0;

VG2 = verbrückende Gruppe ist bei jedem Auftreten unabhängig voneinander ausgewählt aus der Gruppe bestehend aus $CR^{**}_2$, NR**, O, S und einer C-C-Einfachbindung, wobei nicht zwei Einheiten VG2 gleichzeitig gleich einer C-C-Einfachbindung sind;

E ist NR**,

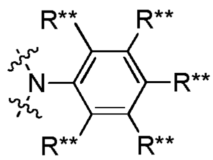

,

O, S;

G ist C wenn o = 1 und gleichzeitig m = 1; ist CR** wenn o = 0 und gleichzeitig m = 1; ist CR**, $CR^{**}_2$, wenn o = 1 und gleichzeitig m = 0; ist R* wenn o = 0 und gleichzeitig m = 0; ist CR**, $CR^{**}_2$, N, NR* wenn m = 0 und gleichzeitig VG1 eine C-C-Einfachbindung ist;

J ist C wenn m = 1; ist CR**, $CR^{**}_2$, NR** wenn m = 0;

L ist CR*** wenn n = 0; ist CR**, C (bei kovalenter Bindung zu VG2 und/oder zu M) wenn n = 1;

R*** ist R** oder ist ausgewählt aus den folgenden Einheiten, wobei maximal zwei der Reste R*** gleichzeitig gleich einer der folgenden Einheiten sind:

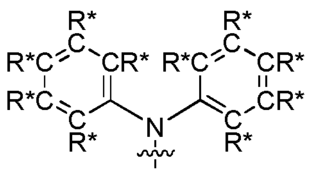

,

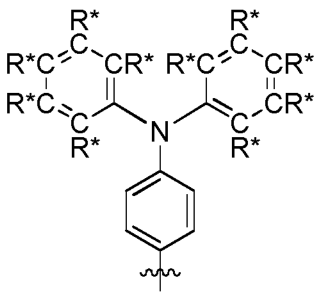

,

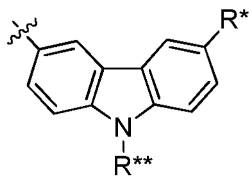

,

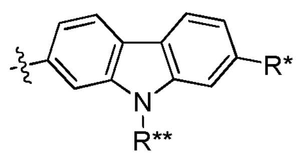

,

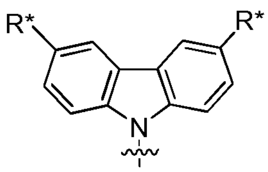

,

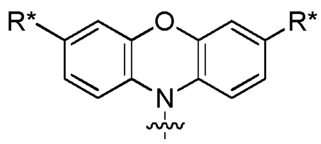

,

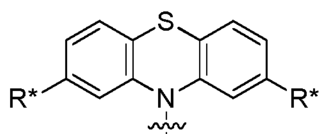

,

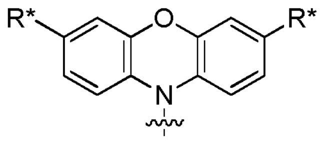

,

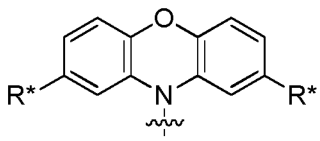

;

R** ist bei jedem Auftreten unabhängig voneinander entweder ein Rest R*, oder kennzeichnet eine chemische Bindung an einen Separator S, wobei genau ein R** eine chemische Bindung an einen Separator S kennzeichnet.

R* ist wie oben definiert.

In einer weiteren Ausführungsform der Beschreibung weist die Einheit AF2 eine Struktur der Formel 5 auf oder

hat eine Struktur der Formel 5

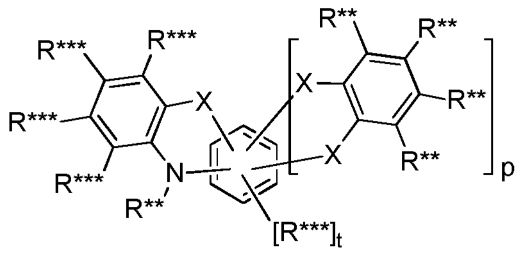

**Formel 5**

wobei in Formel 4 bedeutet:

p ist 0 oder 1;

t = 4 - 2p;

X ist $CR^{**}_2$, $NR^{**}$, Sauerstoff, Schwefel, eine direkte Bindung, wobei maximal zwei Platzhalter X gleichzeitig eine direkte Bindung sind, wobei diese nicht Bestandteil desselben Rings sind;

und im Übrigen die für Formel 4 gegebenen Definitionen gelten.

In einer weiteren Ausführungsform der Beschreibung weist die Einheit AF2 des organischen Moleküls eine Struktur der Formel 5A1-5A7 auf oder hat eine Struktur der Formel 5A1-5A7

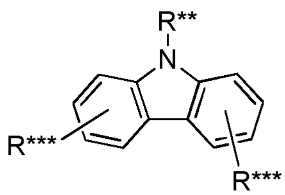

**Formel 5A1**

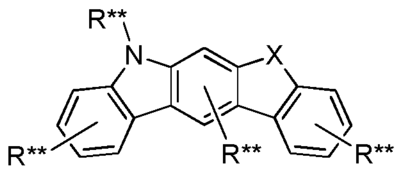

**Formel 5A2**

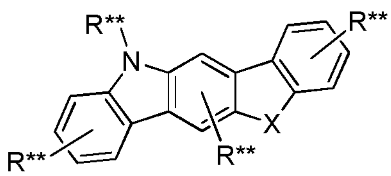

**Formel 5A3**

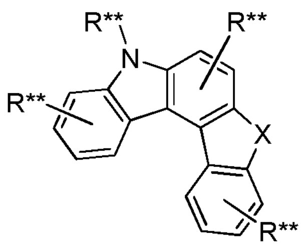

**Formel 5A4**

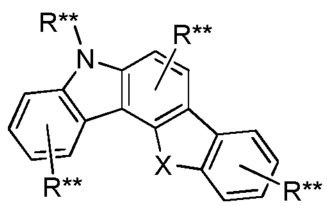

**Formel 5A5**

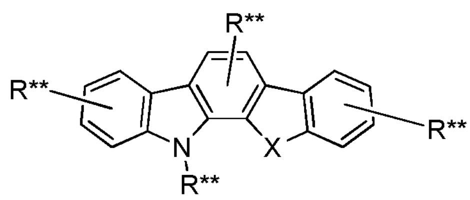

**Formel 5A6**

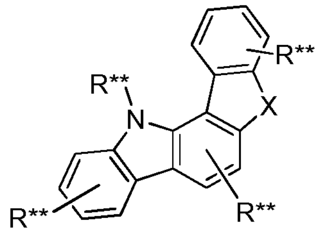

**Formel 5A7**

wobei in Formel 5A1-5A7 gilt:

X ist $C(R^{**})_2$, NR**, Sauerstoff, Schwefel;

und im Übrigen die für Formel 4 gegebenen Definitionen gelten.

In einer weiteren Ausführungsform der Beschreibung weist das organische Molekül eine Struktur der Formel 6 auf oder hat eine Struktur der Formel 6;

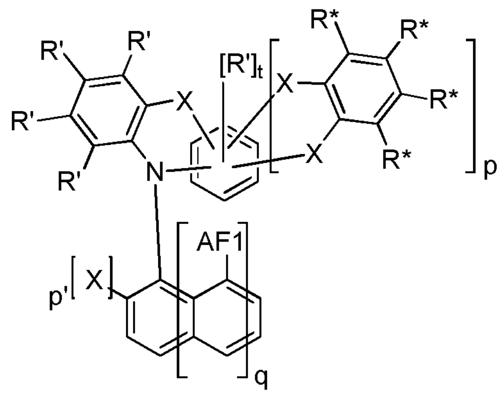

**Formel 6**

X = AF1, $CH_2AF1$, oder

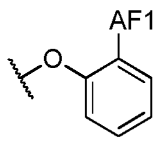

p = 0 oder 1,
p' = 0 oder 1,
q = 0 oder 1,
und wobei p' ≠ q;
t = 4 - 2p;
AF1 eine Struktur der Formel 1b oder eine Struktur der Unterformeln 1.1 bis 1.23 aufweist; R' ist R* oder ist ausgewählt aus den folgenden Einheiten, wobei maximal zwei der Reste R' gleichzeitig gleich einer der folgenden Einheiten sind:

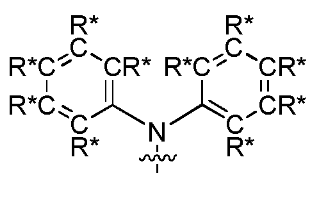 , 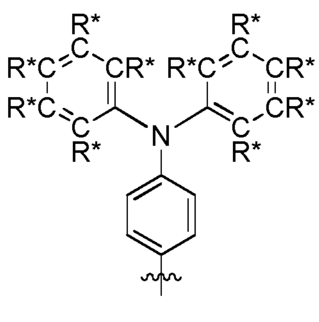 , 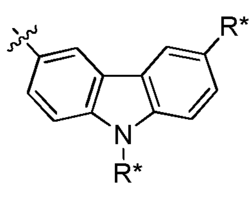 , 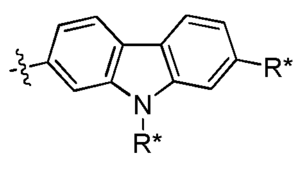 ,

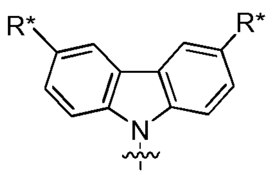 , 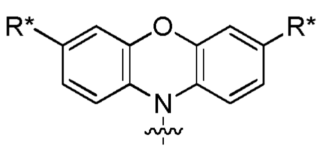 , 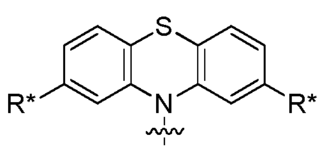 ,

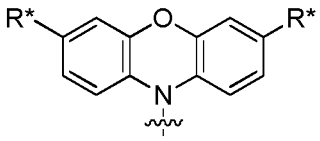

,

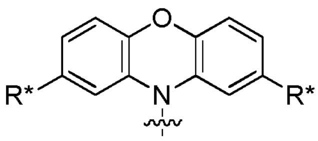

;

und im Übrigen die die für Formel 4 gegebenen Definitionen gelten.

In einer weiteren Ausführungsform der Beschreibung weist das organische Molekül eine Struktur der Formel 7 auf oder hat eine Struktur der Formel 7;

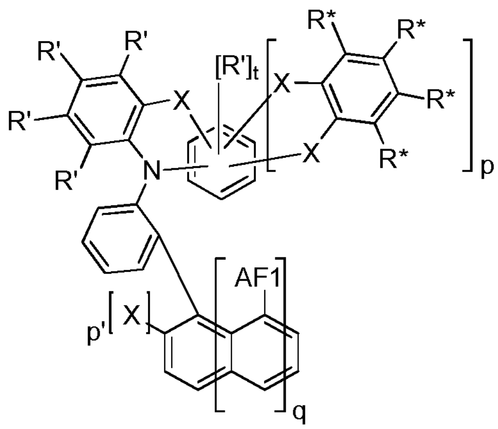

**Formel 7**

X=AF1, $CH_2$AF1, oder

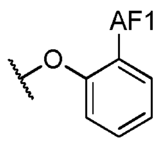

p = 0 oder 1,
p' = 0 oder 1,
q = 0 oder 1,
t = 4 - 2p;
und wobei p' ≠ q;
AF1 eine Struktur der Formel 1b oder eine Struktur der Unterformeln 1.1 bis 1.23 aufweist; R' ist R* oder ist ausgewählt aus den folgenden Einheiten, wobei maximal zwei der Reste R' gleichzeitig gleich einer der folgenden Einheiten sind:

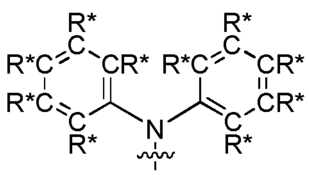

,

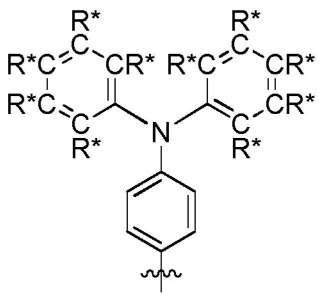

,

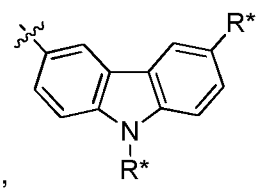

,

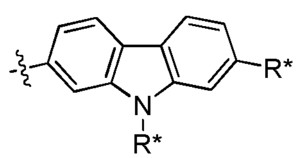

,

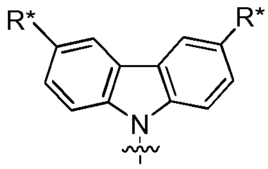 , 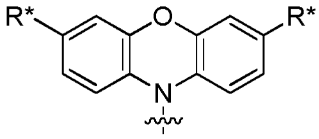 , 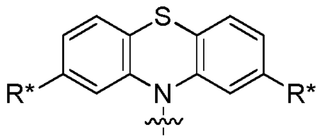 ,

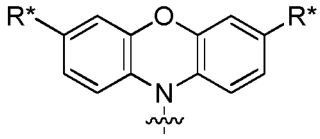 , 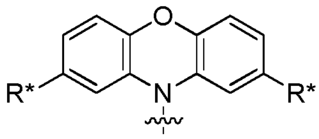 ;

und im Übrigen die die für Formel 4 gegebenen Definitionen gelten.

In einer weiteren Ausführungsform der Beschreibung weist das organische Molekül eine Struktur der Formel 8 auf oder hat eine Struktur der Formel 8;

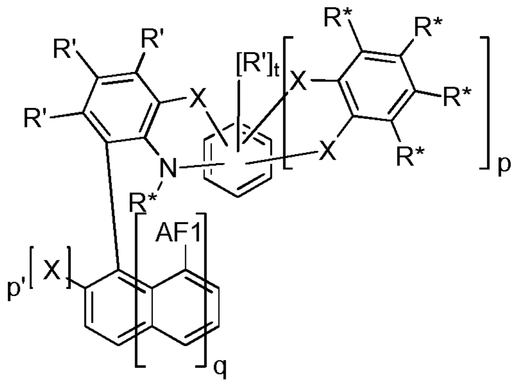

**Formel 8**

X=AF1, $CH_2$AF1, oder

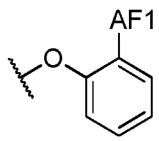

p = 0 oder 1,
p' = 0 oder 1,
q = 0 oder 1,
und wobei p' ≠ q;
t = 4 - 2p;
AF1 eine Struktur der Formel 1b oder eine Struktur der Unterformeln 1.1 bis 1.23 aufweist; R' ist R* oder ist ausgewählt aus den folgenden Einheiten, wobei maximal zwei der Reste R' gleichzeitig gleich einer der folgenden Einheiten sind:

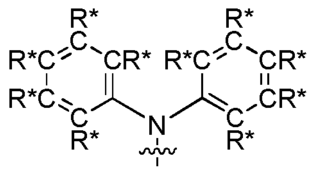,
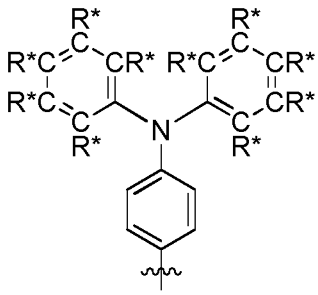,
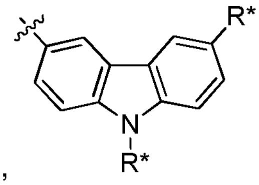,
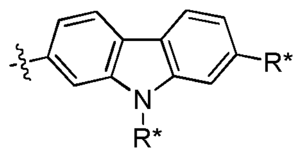,

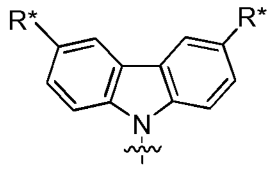,
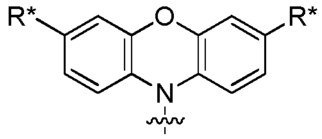,
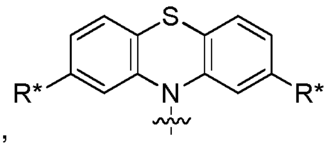,

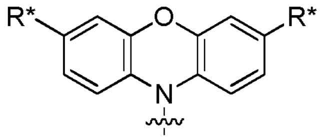,
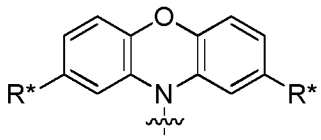;

und im Übrigen die die für Formel 4 gegebenen Definitionen gelten.

In einer weiteren Ausführungsform der Beschreibung weist das organische Molekül eine Struktur der Formel 9 auf oder hat eine Struktur der Formel 9;

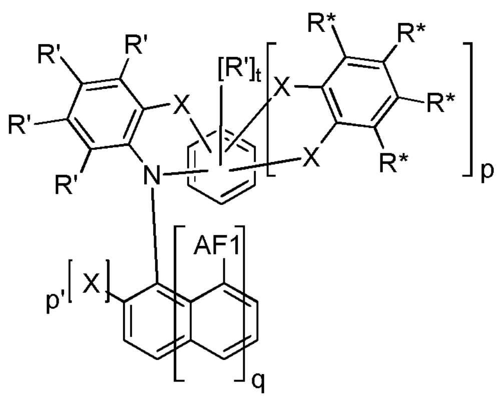

**Formel 9**

X=AF1, $CH_2AF1$, oder

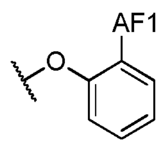

p = 0 oder 1,
p' = 0 oder 1,
q = 0 oder 1,
t = 4 - 2p;
und wobei p' ≠ q;

AF1 eine Struktur der Formel 9b aufweist;

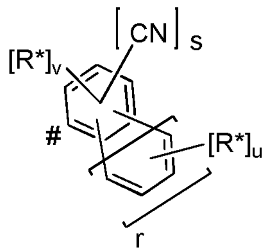

**Formel 9b**

wobei die Anknüpfungsposition für die chemische Einheit AF1 durch den Platzhalter # gekennzeichnet ist,
r = 0 oder 1,
s = 1 bis 5, für r = 1 ist s = 1 bis 3,
u ist 1, 2, 3 oder 4
v ist 1, 2 oder 3; wobei gilt: u + v = 3 + 4r - q
R' ist R* oder ist ausgewählt aus den folgenden Einheiten, wobei maximal zwei der Reste R' gleichzeitig gleich einer der folgenden Einheiten sind:

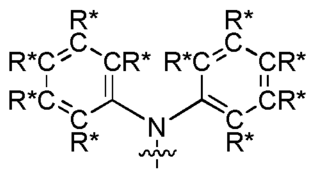 , 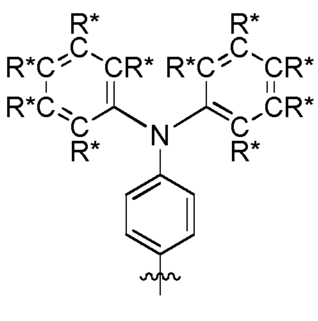 , 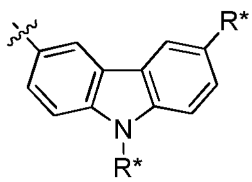 , 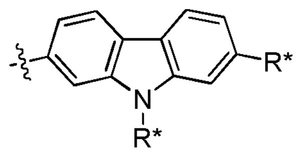 ,

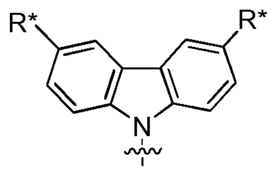 , 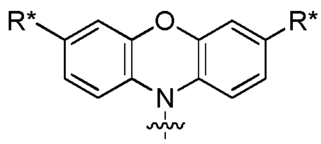 , 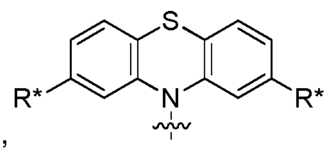 ,

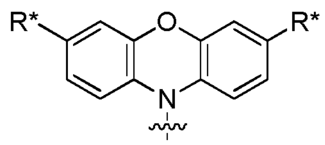 , 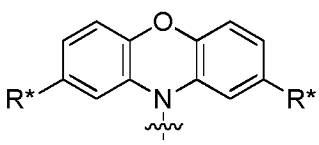 ;

und im Übrigen die die für Formel 4 gegebenen Definitionen gelten.

In einer weiteren Ausführungsform der Beschreibung weist das organische Molekül eine Struktur der Formel 10 auf oder hat eine Struktur der Formel 10;

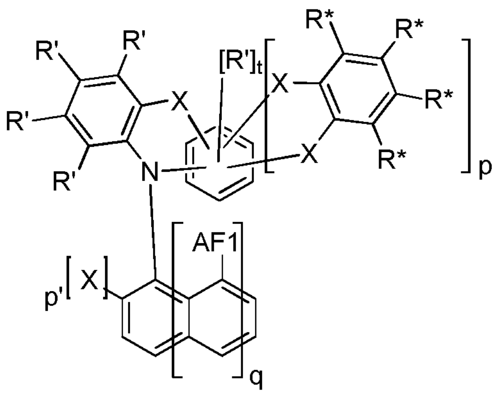

**Formel 10**

X=AF1, $CH_2$AF1, oder

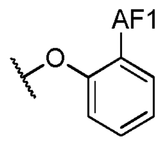

p = 0 oder 1,
p' = 0 oder 1,
q = 0 oder 1,
und wobei p' ≠ q;
t = 4 - 2p;
AF1 eine Struktur der Formel 10b aufweist;

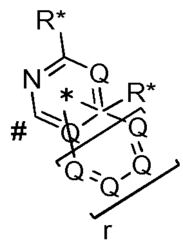

**Formel 10b**

wobei die Anknüpfungsposition für die chemische Einheit AF1 durch den Platzhalter # gekennzeichnet ist,
Q ist N, CR*, wobei mindestens ein Q gleich N ist, und wobei nicht zwei direkt benachbarte Einheiten Q gleichzeitig gleich N sind,
r = 0 oder 1,
R' ist R* oder ist ausgewählt aus den folgenden Einheiten, wobei maximal zwei der Reste R' gleichzeitig gleich einer der folgenden Einheiten sind:

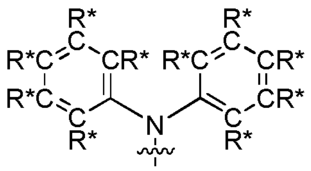 , 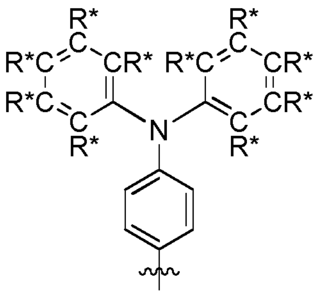 , 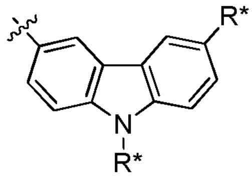 , 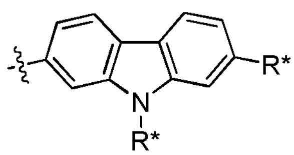 ,

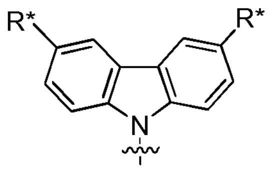 , 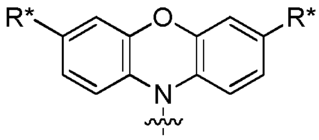 , 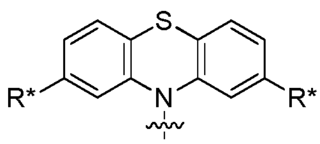 ,

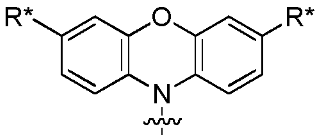 , 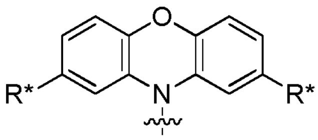 ;

und im Übrigen die die für Formel 4 gegebenen Definitionen gelten.

In einer weiteren Ausführungsform der Beschreibung weist das organische Molekül eine Struktur der Formel 11 auf oder hat eine Struktur der Formel 11;

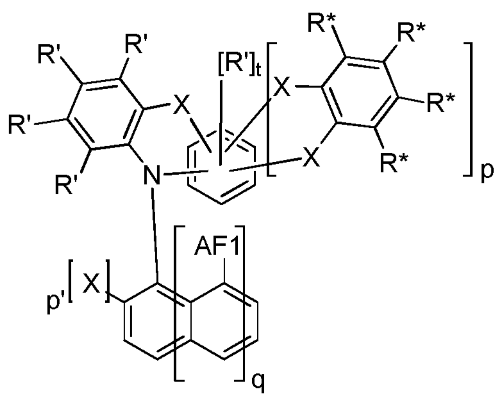

**Formel 11**

X=AF1, $CH_2AF1$, oder

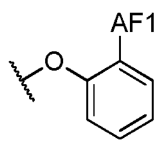

p = 0 oder 1,
p' = 0 oder 1,
q = 0 oder 1,
und wobei p' ≠ q;
t = 4 - 2p;

AF1 eine Struktur der Formel 11b aufweist;

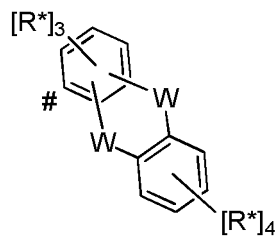

**Formel 11b**

wobei die Anknüpfungsposition für die chemische Einheit AF1 durch den Platzhalter # gekennzeichnet ist, W ist ausgewählt aus der Gruppe bestehend aus

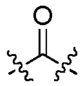, 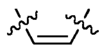, 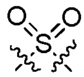,

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;
R' ist R* oder ist ausgewählt aus den folgenden Einheiten, wobei maximal zwei der Reste R' gleichzeitig gleich einer der folgenden Einheiten sind:

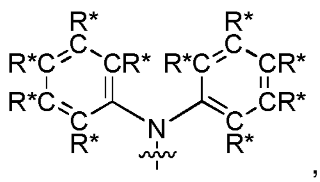, 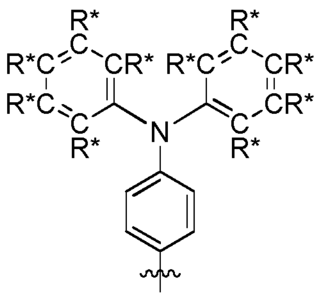, 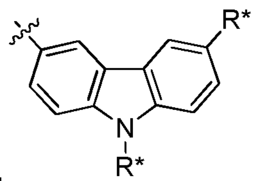, 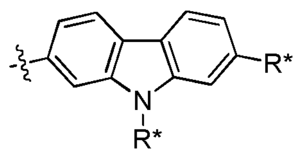,

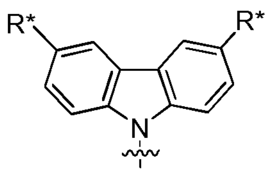, 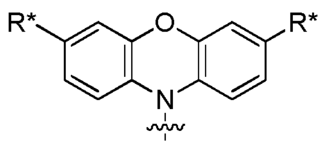, 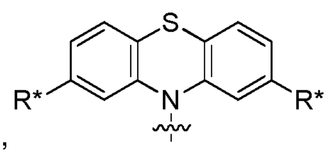,

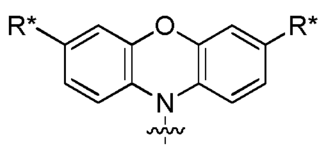, 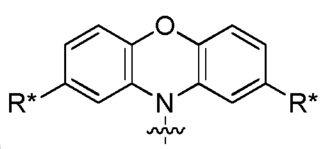;

und im Übrigen die die für Formel 4 gegebenen Definitionen gelten.

In einer weiteren Ausführungsform der Beschreibung weist das organische Molekül eine Struktur der Formel 12 auf oder hat eine Struktur der Formel 12;

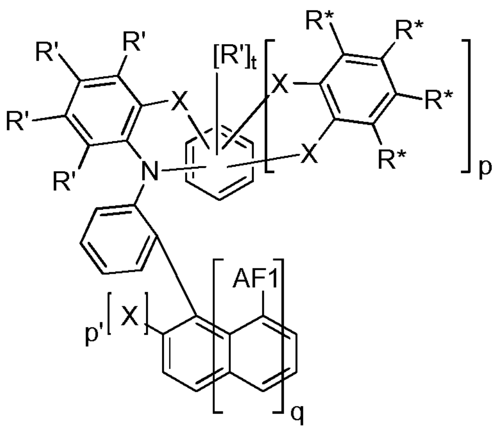

**Formel 12**

X=AF1, $CH_2$AF1, oder

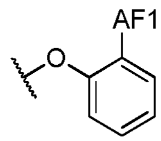

p = 0 oder 1,

p' = 0 oder 1,

q = 0 oder 1,

und wobei p' ≠ q;

t = 4 - 2p;

AF1 eine Struktur der Formel 9b aufweist;

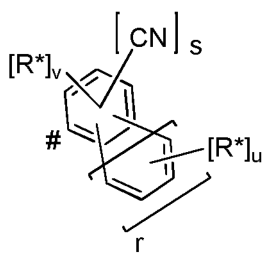

**Formel 9b**

wobei die Anknüpfungsposition für die chemische Einheit AF1 durch den Platzhalter # gekennzeichnet ist,

r = 0 oder 1,

s = 1 bis 5, für r = 1 ist s = 1 bis 3,

u ist 1, 2, 3 oder 4

v ist 1, 2 oder 3; wobei gilt: u + v = 3 + 4r - q

R' ist R* oder ist ausgewählt aus den folgenden Einheiten, wobei maximal zwei der Reste R' gleichzeitig gleich einer der folgenden Einheiten sind:

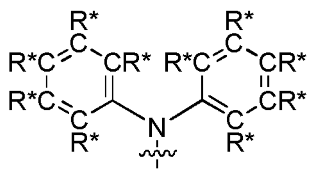
,
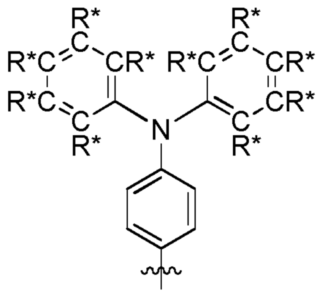
,
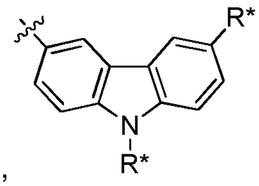
,
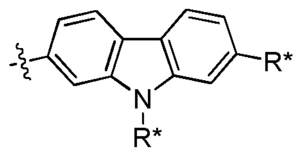
,
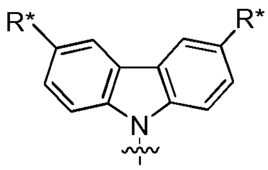
,
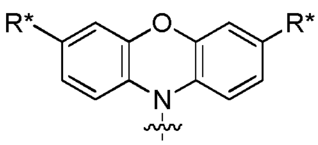
,
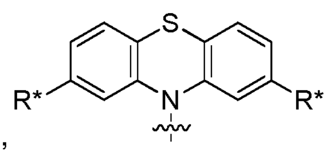
,
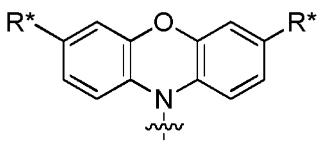
,
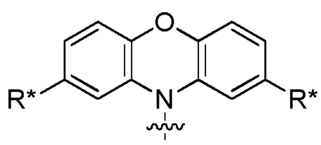
;

und im Übrigen die die für Formel 4 gegebenen Definitionen gelten.

In einer weiteren Ausführungsform der Beschreibung weist das organische Molekül eine Struktur der Formel 13 auf oder hat eine Struktur der Formel 13;

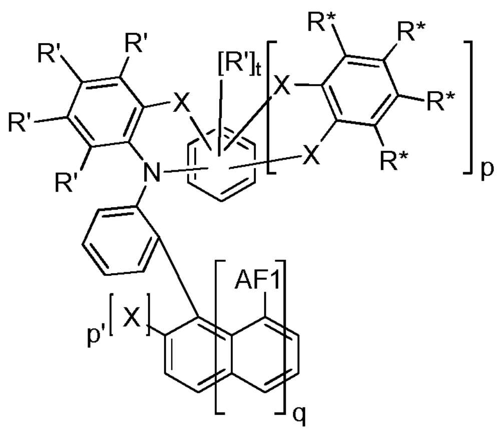

**Formel 13**

X=AF1, $CH_2AF1$, oder

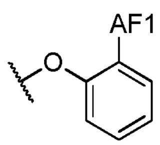

p = 0 oder 1,
p' = 0 oder 1,
q = 0 oder 1,
und wobei p' ≠ q;
t = 4 - 2p;
AF1 eine Struktur der Formel 10b aufweist;

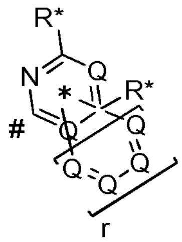

**Formel 10b**

wobei die Anknüpfungsposition für die chemische Einheit AF1 durch den Platzhalter # gekennzeichnet ist,
Q ist N, CR*, wobei mindestens ein Q gleich N ist, und wobei nicht zwei direkt benachbarte Einheiten Q gleichzeitig gleich N sind,
r = 0 oder 1,
R' ist R* oder ist ausgewählt aus den folgenden Einheiten, wobei maximal zwei der Reste R' gleichzeitig gleich einer der folgenden Einheiten sind:

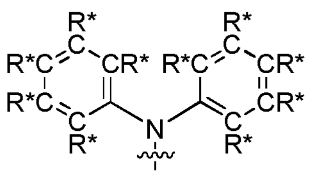 , 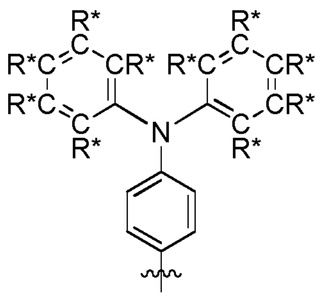 , 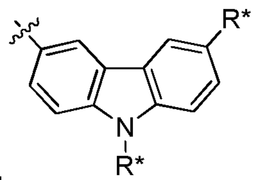 , 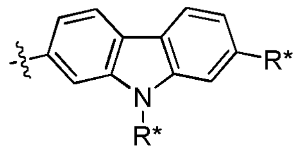 ,

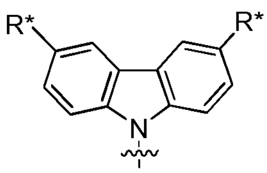 , 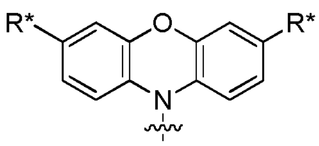 , 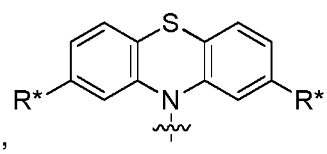 ,

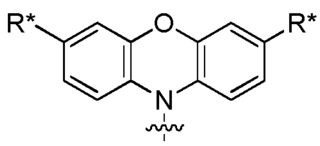 , 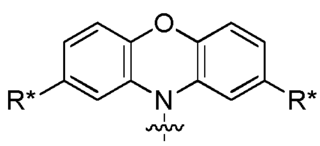 ;

und im Übrigen die die für Formel 4 gegebenen Definitionen gelten.

In einer weiteren Ausführungsform der Beschreibung weist das organische Molekül eine Struktur der Formel 14 auf oder hat eine Struktur der Formel 14;

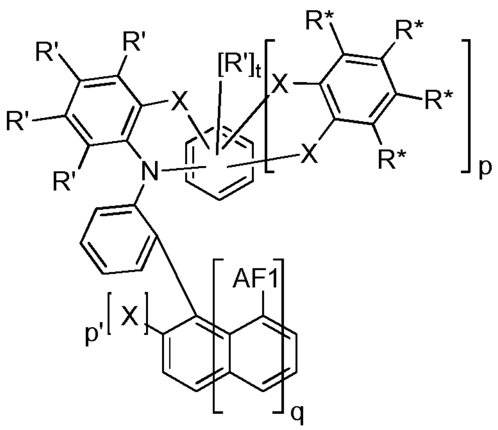

**Formel 14**

X=AF1, $CH_2AF1$, oder

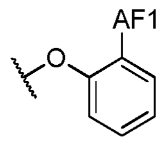

p = 0 oder 1,
p' = 0 oder 1,
q = 0 oder 1,
und wobei p' ≠ q;
t = 4 - 2p;
AF1 eine Struktur der Formel 11b aufweist;

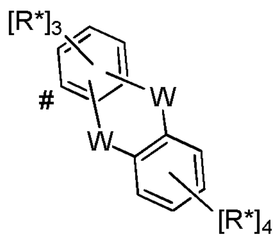

**Formel 11b**

wobei die Anknüpfungsposition für die chemische Einheit AF1 durch den Platzhalter # gekennzeichnet ist,
W ist ausgewählt aus der Gruppe bestehend aus

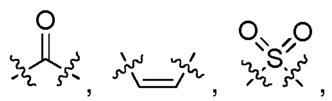

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;
R' ist R* oder ist ausgewählt aus den folgenden Einheiten, wobei maximal zwei der Reste R' gleichzeitig gleich einer der folgenden Einheiten sind:

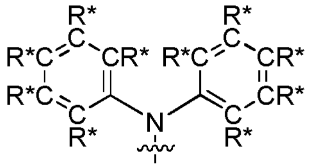 , 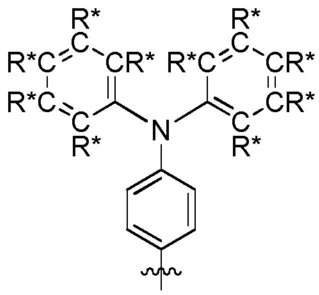 , 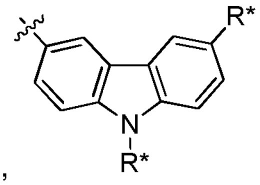 , 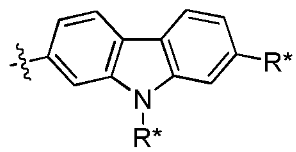 ,

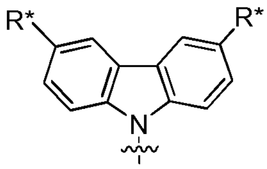 , 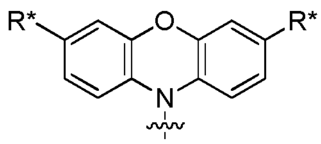 , 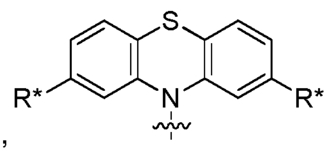 ,

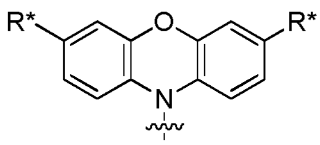 , 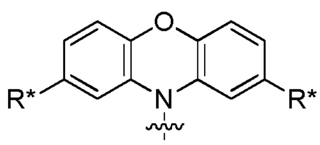 ;

und im Übrigen die die für Formel 4 gegebenen Definitionen gelten.

In einer weiteren Ausführungsform der Beschreibung weist das organische Molekül eine Struktur der Formel 15 auf oder hat eine Struktur der Formel 15;

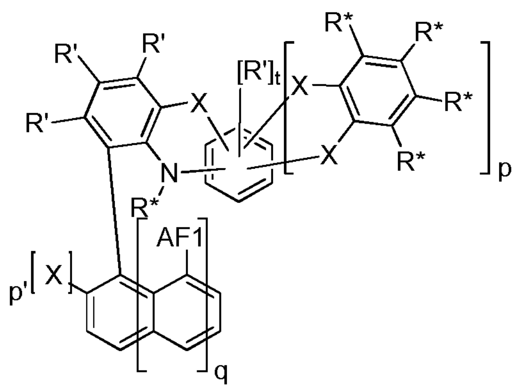

**Formel 15**

X = AF1, $CH_2AF1$, oder

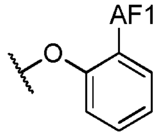

p = 0 oder 1,
p' = 0 oder 1,
q = 0 oder 1,
und wobei p' ≠ q;
t = 4 - 2p;

AF1 eine Struktur der Formel 9b aufweist;

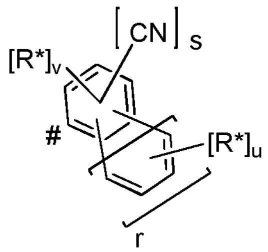

**Formel 9b**

wobei die Anknüpfungsposition für die chemische Einheit AF1 durch den Platzhalter # gekennzeichnet ist,
r = 0 oder 1,
s = 1 bis 5, für r = 1 ist s = 1 bis 3,
u ist 1, 2, 3 oder 4
v ist 1, 2 oder 3; wobei gilt: u + v = 3 + 4r - q
R' ist R* oder ist ausgewählt aus den folgenden Einheiten, wobei maximal zwei der Reste R' gleichzeitig gleich einer der folgenden Einheiten sind:

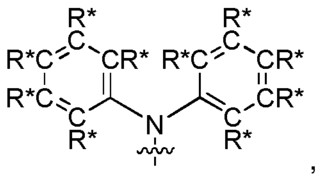 , 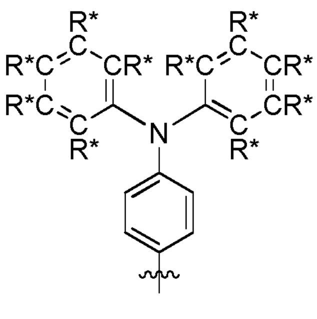 , 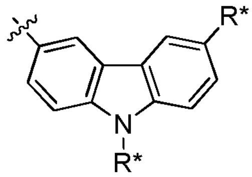 , 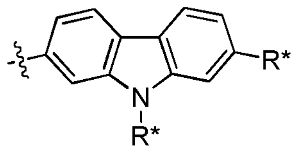 ,

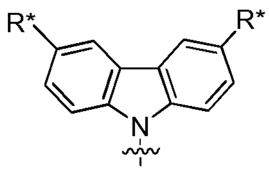 , 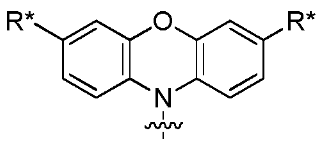 , 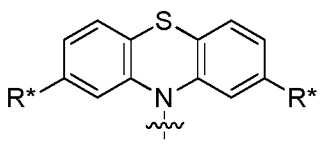 ,

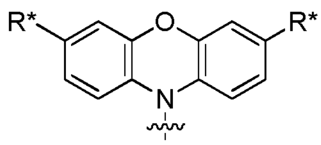 , 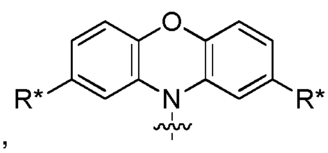 ;

und im Übrigen die die für Formel 4 gegebenen Definitionen gelten.

In einer weiteren Ausführungsform der Beschreibung weist das organische Molekül eine Struktur der Formel 16 auf oder hat eine Struktur der Formel 16;

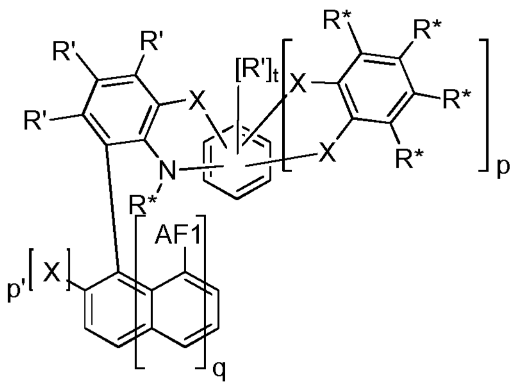

**Formel 16**

X=AF1, $CH_2$AF1, oder

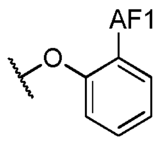

p = 0 oder 1,
p' = 0 oder 1,
q = 0 oder 1,
t = 4 - 2p;
und wobei p' ≠ q;
AF1 eine Struktur der Formel 10b aufweist;

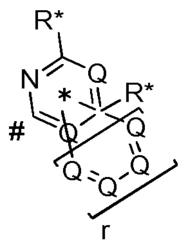

**Formel 10b**

wobei die Anknüpfungsposition für die chemische Einheit AF1 durch den Platzhalter # gekennzeichnet ist,
Q ist N, CR*, wobei mindestens ein Q gleich N ist, und wobei nicht zwei direkt benachbarte Einheiten Q gleichzeitig gleich N sind,
r = 0 oder 1,
R' ist R* oder ist ausgewählt aus den folgenden Einheiten, wobei maximal zwei der Reste R' gleichzeitig gleich einer der folgenden Einheiten sind:

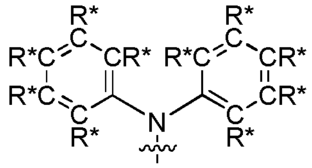, 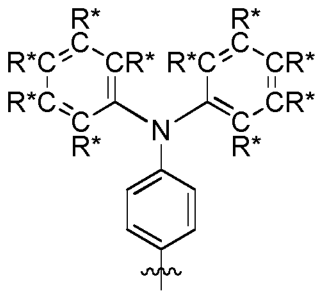, 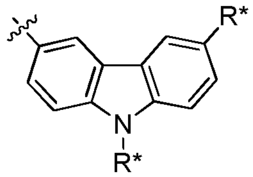, 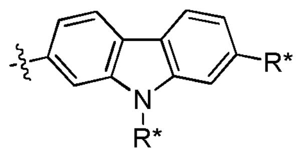,

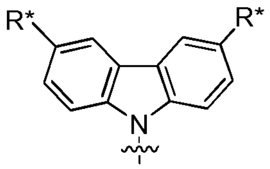, 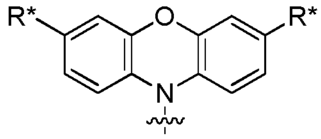, 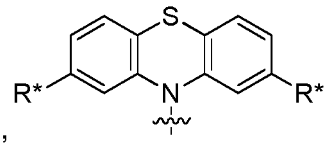,

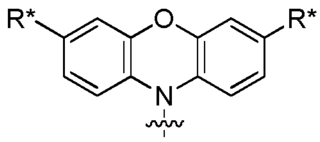, 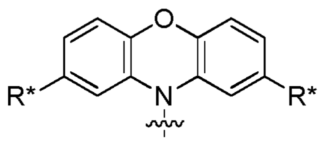;

und im Übrigen die die für Formel 4 gegebenen Definitionen gelten.

In einer weiteren Ausführungsform der Beschreibung weist das organische Molekül eine Struktur der Formel 17 auf oder hat eine Struktur der Formel 17;

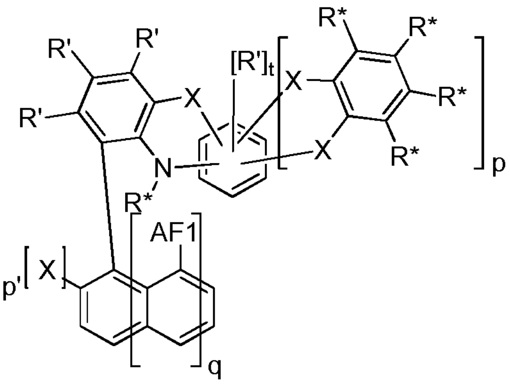

**Formel 17**

X=AF1, $CH_2$AF1, oder

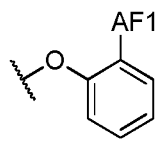

p = 0 oder 1,
p' = 0 oder 1,
q = 0 oder 1,
und wobei p' ≠ q;
t = 4 - 2p;

AF1 eine Struktur der Formel 11b aufweist;

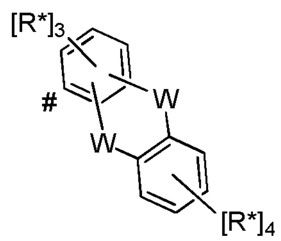

**Formel 11b**

wobei die Anknüpfungsposition für die chemische Einheit AF1 durch den Platzhalter # gekennzeichnet ist,
W ist ausgewählt aus der Gruppe bestehend aus

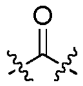, 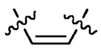, 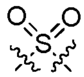,

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;
R' ist R* oder ist ausgewählt aus den folgenden Einheiten, wobei maximal zwei der Reste R' gleichzeitig gleich einer der folgenden Einheiten sind:

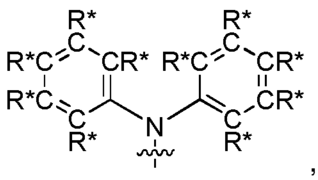, 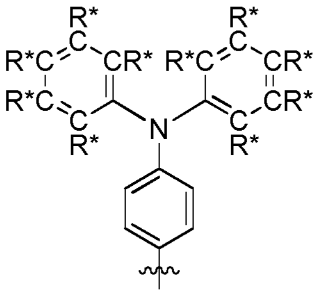, 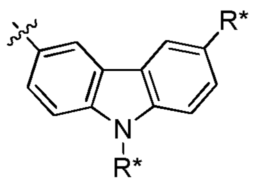, 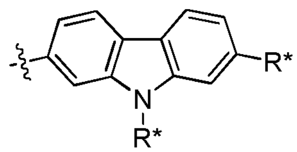,

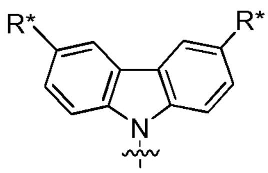, 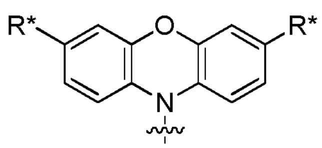, 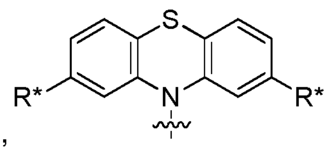,

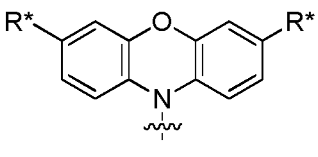, 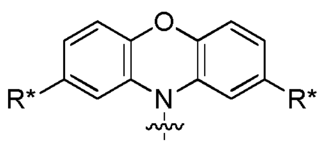;

und im Übrigen die die für Formel 4 gegebenen Definitionen gelten.

Erfindungsgemäße organische Moleküle sind in Tabelle 1 dargestellt die sich durch Kombination aus chemischen Einheiten AF1 und AF2, dem Separator und der Festlegung der Verknüpfung ergeben. Weitere organische Moleküle können durch Kombination der genannten Moleküleinheiten erhalten werden. Die Benennung der Moleküle erfolgt nach dem Schema AF-S-AF (nicht erfindungsgemäße Moleküle sind mit * markiert).

**Tabelle 1:** Erfindungsgemäße organische Moleküle nach dem Schema AF-S-AF. In Klammern sind die Werte für ΔHOMO, ΔLUMO und Gap angegeben

| | | | |
|---|---|---|---|
| 41-S-46 (2.68 1.75 1.14) | 41-S-111 (2.18 1.00 1.89) | 41-S-118 (1.47 1.28 1.61) | 41-S-150 (3.01 1.88 1.01) |
| 41-S-153 (2.95 1.98 0.91) | 41-S-198 (1.90 1.38 1.51) | 41-S-222 (1.18 0.95 1.94) | 41-S-233 (1.80 1.53 1.36) |
| 55-S-36 (0.95 2.03 1.23) | 55-S-43 (1.37 1.89 1.38) | 55-S-46 (1.96 1.41 1.86) | 55-S-150 (2.29 1.53 1.73) |
| 55-S-153 (2.23 1.63 1.63) | 55-S-198 (1.19 1.03 2.23) | 55-S-233 (1.08 1.18 2.08) | 77-S-46 (2.56 1.40 1.26) |
| 77-S-118 (1.35 0.92 1.73) | 77-S-150 (2.89 1.52 1.13) | 77-S-153 (2.83 1.63 1.03) | 77-S-198 (1.79 1.02 1.63) |
| 77-S-233 (1.68 1.17 1.48) | 78-S-46 (2.74 1.45 1.08) | 78-S-118 (1.53 0.97 1.56) | 78-S-150 (3.07 1.58 0.95) |
| 78-S-198 (1.96 1.07 1.46) | 78-S-233 (1.86 1.23 1.30) | 106-S-43 (0.94 1.82 1.80) | 106-S-46 (1.54 1.34 2.28) |
| 106-S-57 (1.21 2.36 1.26) | 106-S-150 (1.87 1.46 2.15) | 106-S-153 (1.80 1.57 2.05) | 111-S-150 (0.83 0.88 3.20)* |
| 118-S-57 (0.89 1.49 1.59)* | 222-S-43 (0.91 1.28 1.84)* | 222-S-46 (1.50 0.81 2.32)* | 222-S-57 (1.17 1.82 1.30)* |
| 222-S-150 (1.83 0.93 2.19)* | 222-S-153 (1.77 1.03 2.09)* | 223-S-36 (1.24 2.49 0.94) | 223-S-43 (1.66 2.34 1.09) |
| 223-S-46 (2.26 1.86 1.56) | 223-S-111 (1.76 1.11 2.32) | 223-S-118 (1.04 1.39 2.04) | 223-S-150 (2.59 1.99 1.44) |
| 223-S-153 (2.52 2.09 1.34) | 223-S-198 (1.48 1.49 1.94) | 223-S-233 (1.37 1.64 1.79) | 317-S-43 (1.77 2.15 0.97) |
| 317-S-46 (2.37 1.67 1.45) | 317-S-111 (1.87 0.92 2.20) | 317-S-118 (1.15 1.20 1.93) | 317-S-150 (2.70 1.80 1.33) |
| 317-S-153 (2.63 1.90 1.22) | 317-S-198 (1.59 1.30 1.83) | 317-S-222 (0.86 0.87 2.26) | 317-S-233 (1.48 1.45 1.68) |
| 328-S-36 (0.88 2.05 1.30) | 328-S-43 (1.30 1.90 1.45) | 328-S-46 (1.89 1.42 1.92) | 328-S-57 (1.57 2.44 0.91)* |
| 328-S-150 (2.23 1.55 1.80) | 328-S-153 (2.16 1.65 1.70) | 328-S-198 (1.12 1.05 2.30) | 328-S-233 (1.01 1.20 2.15) |
| 337-S-46 (2.45 1.94 1.37) | 337-S-111 (1.95 1.19 2.12) | 337-S-118 (1.24 1.46 1.85) | 337-S-150 (2.78 2.07 1.24) |
| 337-S-153 (2.71 2.17 1.14) | 337-S-198 (1.67 1.56 1.75) | 337-S-222 (0.95 1.13 2.18) | 337-S-233 (1.57 1.71 1.59) |
| 425-S-111 (2.73 0.84 1.34) | 425-S-118 (2.01 1.12 1.07) | 425-S-198 (2.45 1.22 0.97) | 432-S-46 (1.30 1.15 2.52) |
| 432-S-57 (0.97 2.17 1.50)* | 432-S-150 (1.63 1.28 2.39) | 432-S-153(1.56 1.38 2.29) | 433-S-43 (0.99 1.62 1.76) |
| 433-S-46 (1.58 1.14 2.24) | 433-S-57 (1.26 2.16 1.22)* | 433-S-150 (1.92 1.27 2.11) | 433-S-153 (1.85 1.37 2.01) |

Beispiele für erfindungsgemäße organische Moleküle (nicht erfindungsgemäße Moleküle sind mit * markiert)

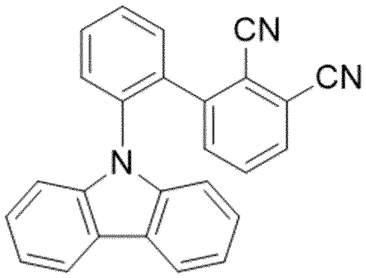

328 - S-a -153

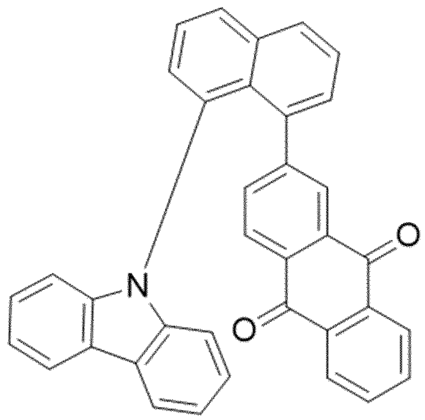

328 - S-c -36

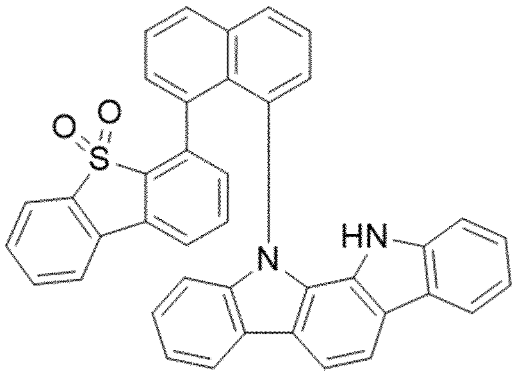

Indolo[2,3-a] 328 - S-c - 198
(Indolo[2,3-a]carbazol - S-c - 198)

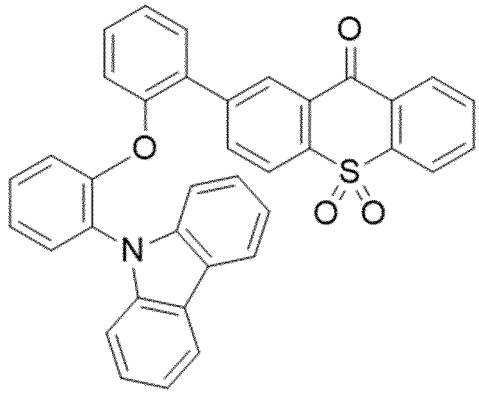

328 - S-e - 43

**Indolocarbazol (INDC) als Donor**

Indolocarbazol (INDC) als Donor

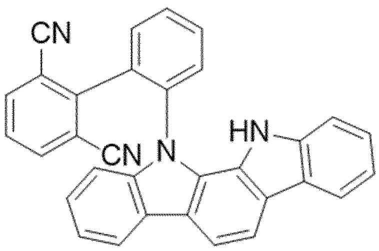

Indolo[2,3-a] 328 - S-a - 150
(Indolo[2,3-a]carbazol - S-a - 150)

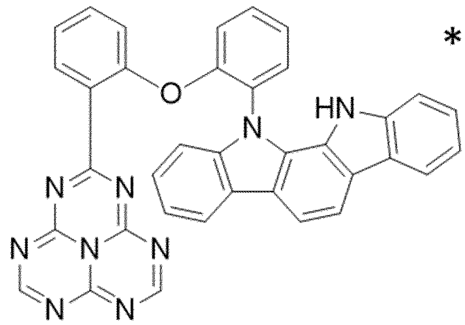

*

Indolo[2,3-a] 328 - S-e - 57
(Indolo[2,3-a]carbazol - S-e - 57)

**Dimethoxycarbaxol (DiMC) als Donor**

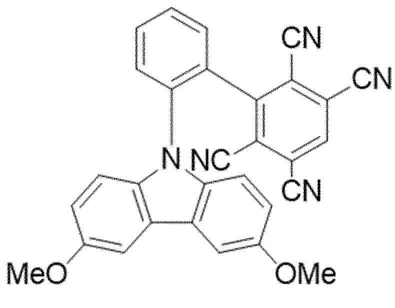

3,6-Dimethoxy 328 - S-a – 150-1
(3,6-Dimethoxycarbazol - S-a - 150-1)

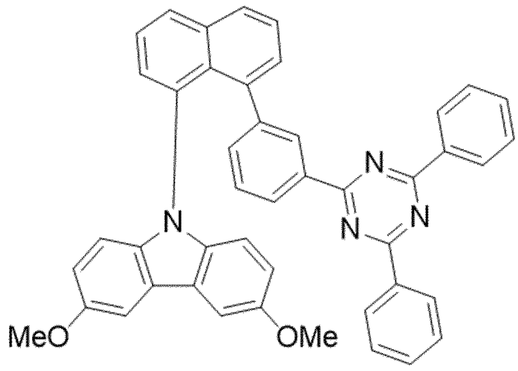

3,6-Dimethoxy 328 - S-c - 233-1
(3,6-Dimethoxycarbazol - S-c - 233-1)

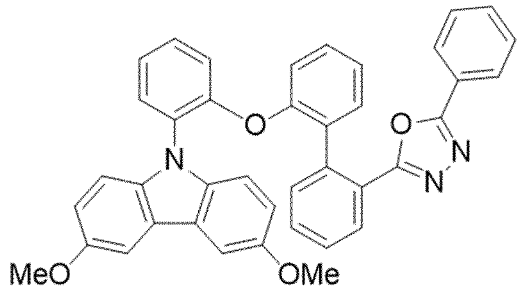

3,6-Dimethoxy 328 - S-e - 118
(3,6-Dimethoxycarbazol - S-e - 118)

**Diphenyltriazin (233) als Akzeptor**

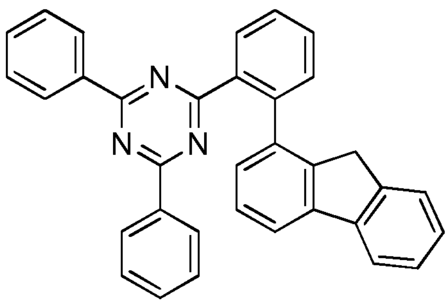

106 - S-a - 233

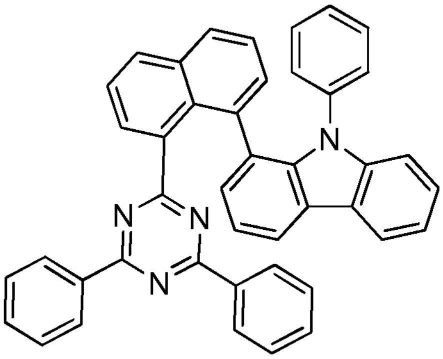

55 - S-c - 233

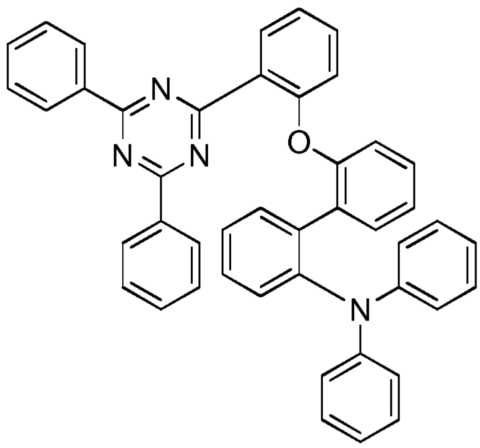

317 - S-e - 233

**Triphenyltriazin (233-1) als Akzeptor**

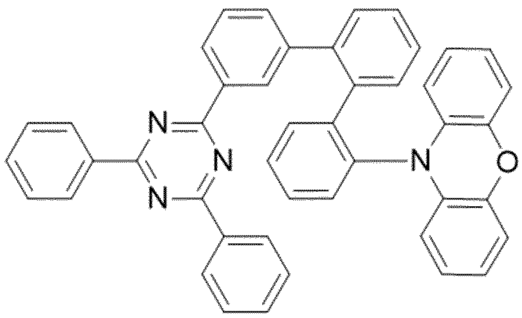

78 - S-a - 233-1

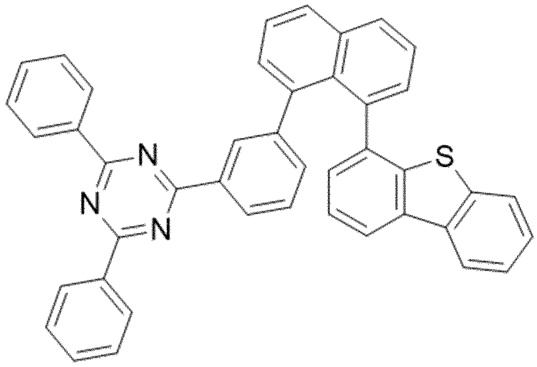

433 - S-c - 233-1

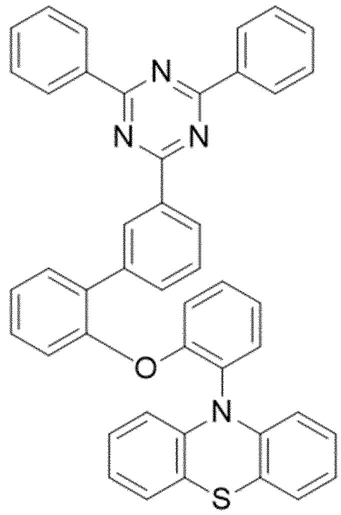

77-1 - S-e - 233-1

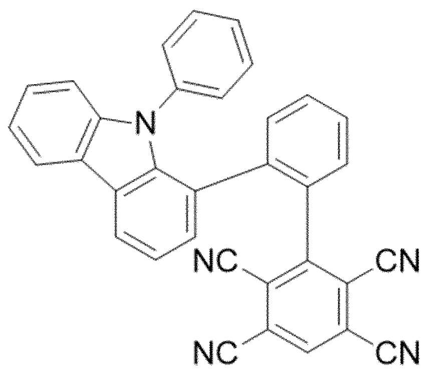

55 - S-a - 150-1

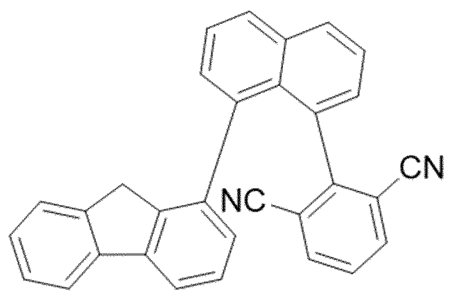

106 - S-c - 150

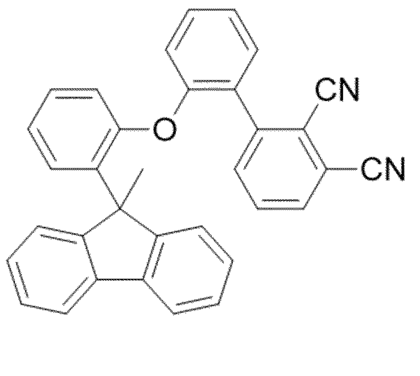

106-1 - S-e - 153

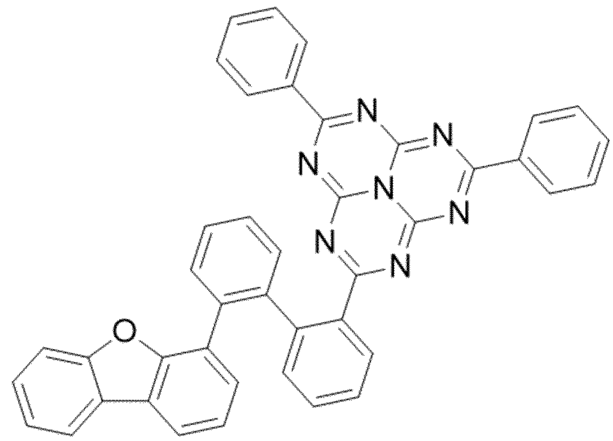

432 - S-a - 57-1*

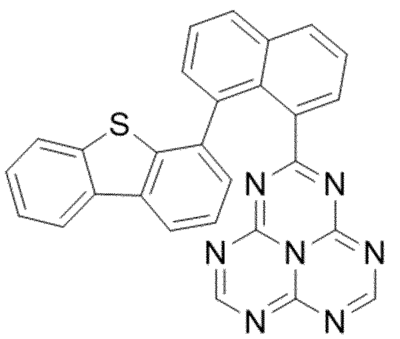

433 - S-c- 57*

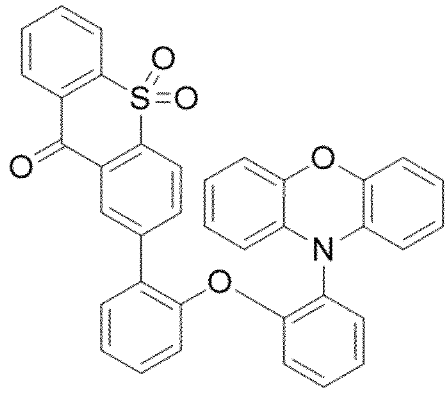

41 - S-e - 43

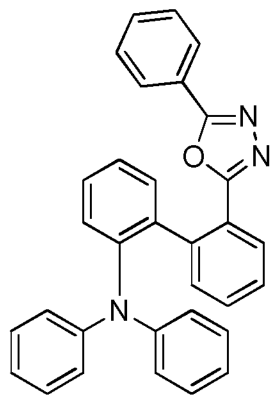

223 - S-a - 118

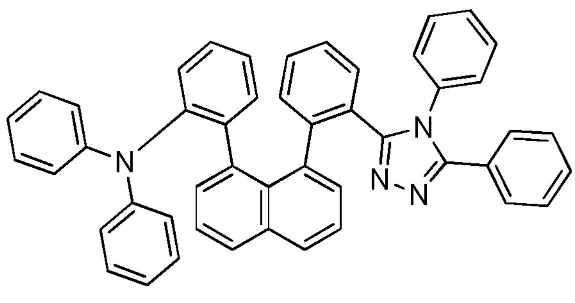

317 - S-c - 222-1

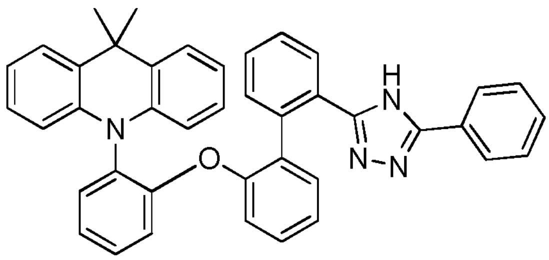

337 - S-e - 222

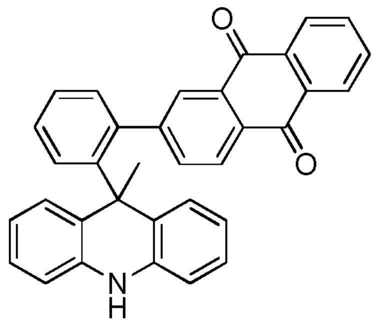

337-1 - S-a - 36

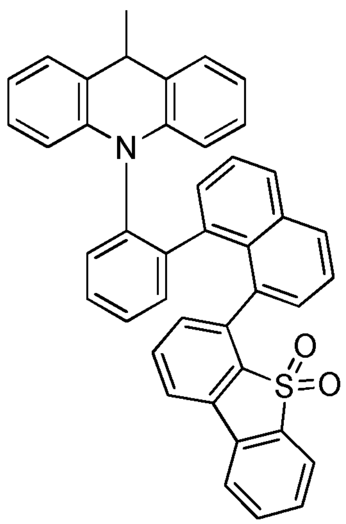

337-2 - S-c - 198

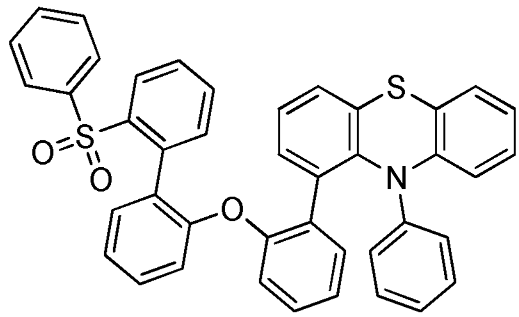

77 - S-e - 111

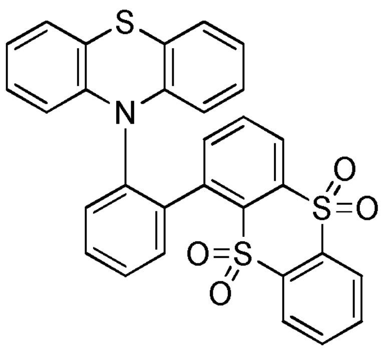

77-1 - S-a - 46

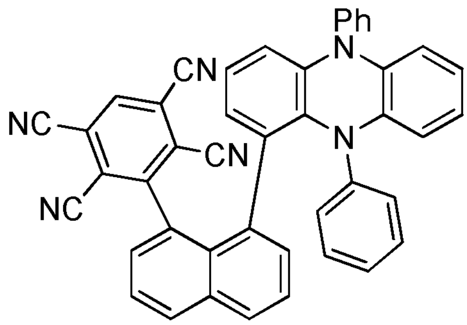

425 - S-c - 150-1

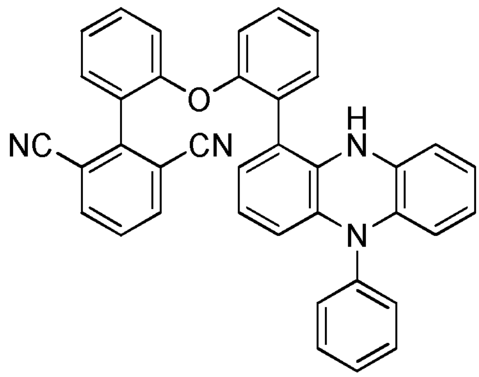

425-1 - S-e - 150

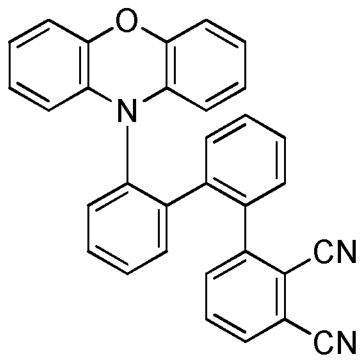

78 - S-a - 153

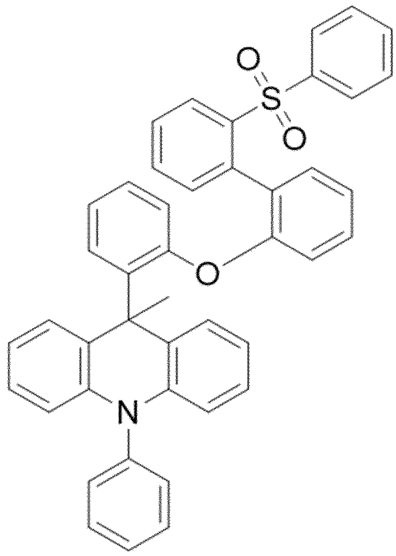

337-2 - S-e - 111

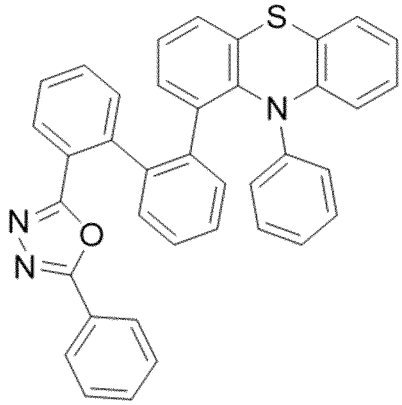

77-1 - S-a - 118

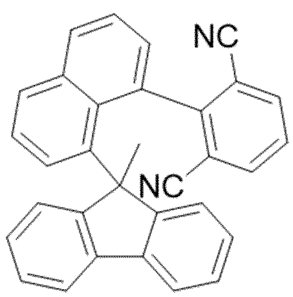

106-1 - S-c - 150

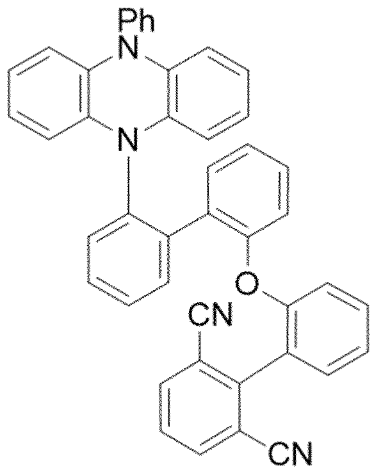

425 - S-e - 150

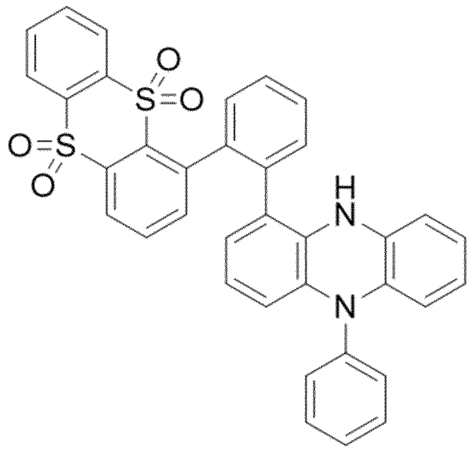

425-1 - S-a - 46

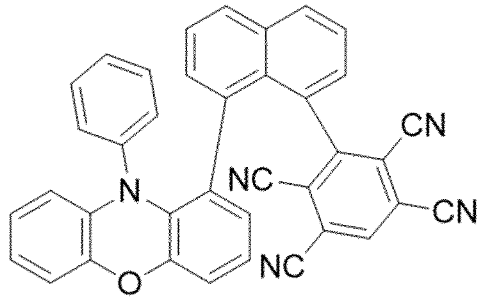

78 - S-c - 150-1

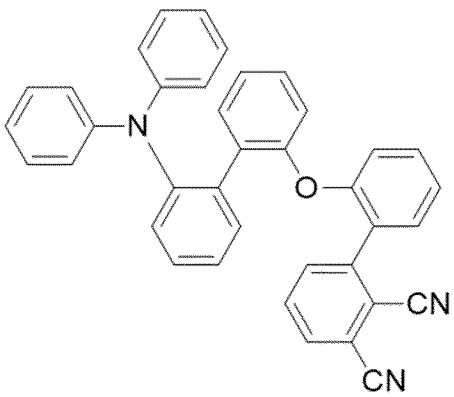

317 - S-e - 153

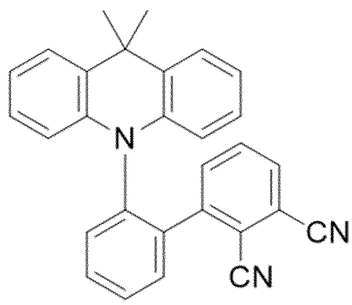

337 - S-a - 153

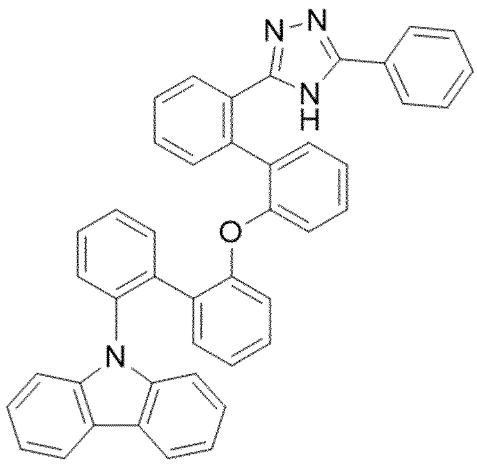

55 - S-e - 222

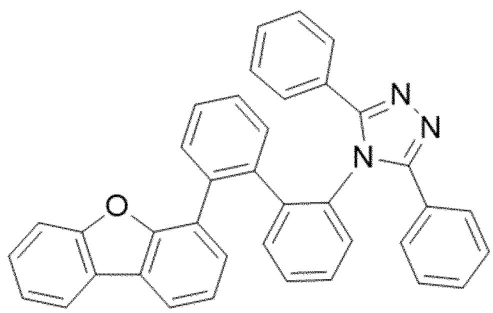

432 - S-a - 222-1

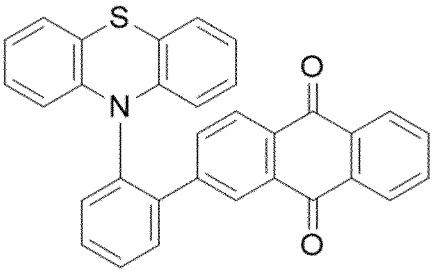

77-1 - S-a - 36

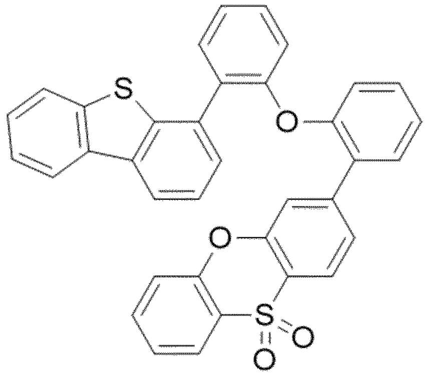

433 - S-e - 43

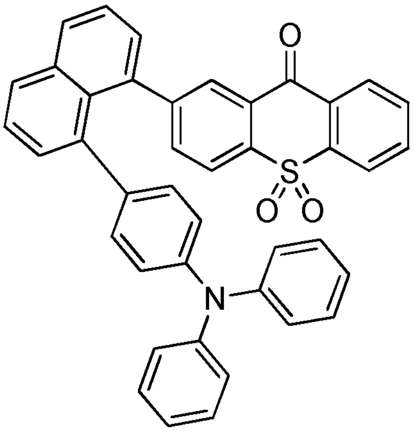

317 - S-c - 43

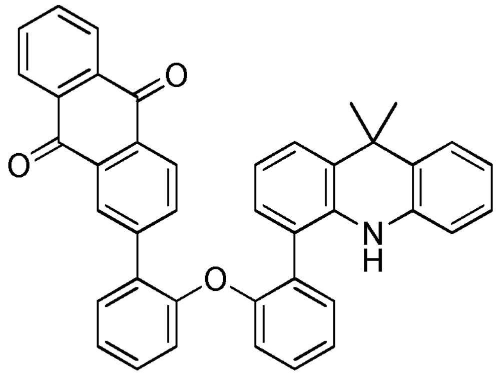

337 - S-e - 36

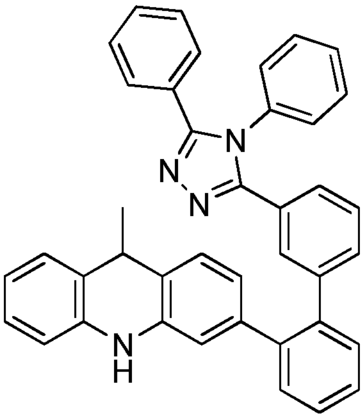

337-1 - S-a - 222-1

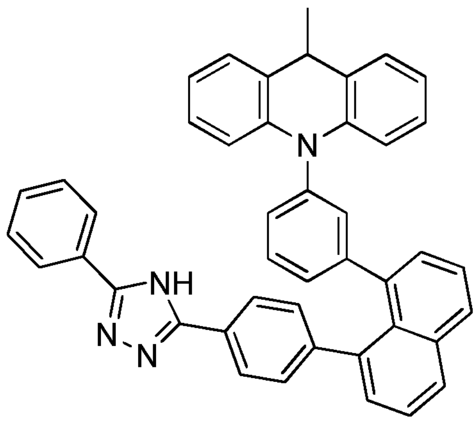

337-2 - S-c - 222

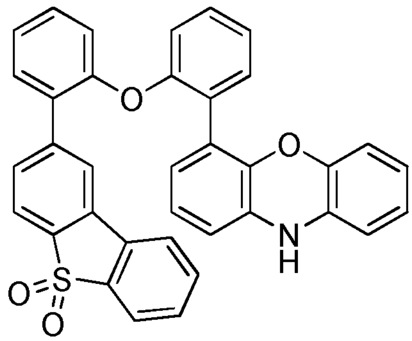

41 - S-e - 198

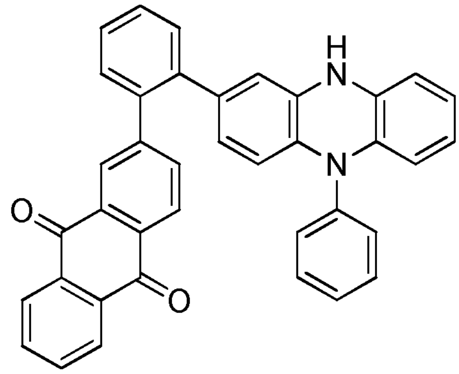

425-1 - S-a - 36

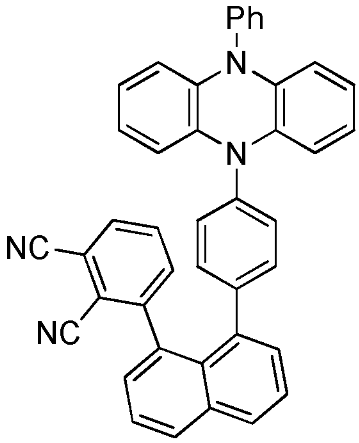

425 - S-c - 153

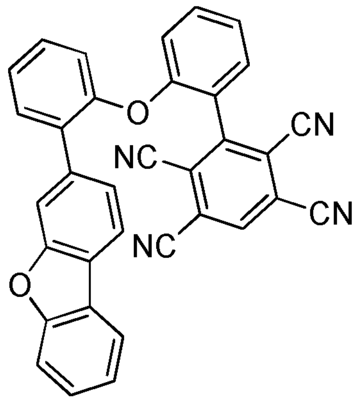

432 - S-e - 150-1

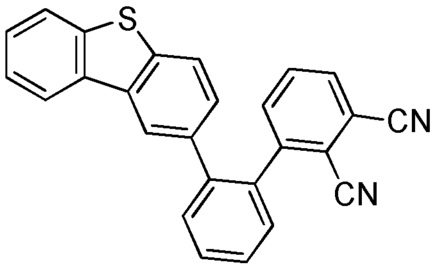

433 - S-a - 153

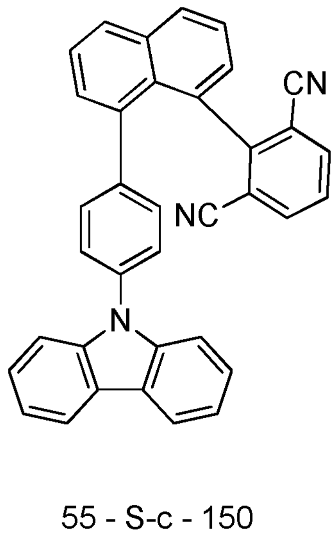

55 - S-c - 150

In einer Ausführungsform der Erfindung werden an die chemisch substituierbaren Positionen der so erhaltenen organischen Moleküle weitere Reste R angefügt, um die Löslichkeit der Emitter zu steigern und/oder die Polymerisierbarkeit zu ermöglichen ohne dabei die elektronischen Eigenschaften des Moleküls signifikant zu verändern, sodass

auch bei Verwendung von R ein Emitter vorliegt, wobei

jedes R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$,$-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein mono- oder polycyclisches, aliphatisches, aromatisches und/oder benzoannelliertes Ringsystem bilden. In einer Ausführungsform ist das Ringsystem, das gebildet werden kann auf ein monozyklisches aliphatisches Ringsystem mit insgesamt fünf oder sechs Ringgliedern beschränkt.

Die Reste $R^2$ bis $R^9$ sind hierbei so definiert wie bei R* angegeben.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 20 und 21 erhalten werden, die als Polymere in der lichtemittierenden Schicht des optoelektronischen Bauelements Verwendung finden können.

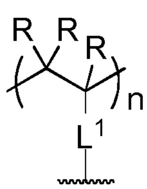
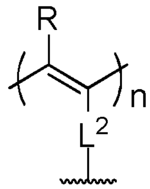

**Formel 20** **Formel 21**

In Formel 20 und 21 stehen L1 und L2 für gleiche oder verschiedene Linkergruppen, die 0 bis 20, insbesondere 1 bis 15, oder 2 bis 10 Kohlenstoffatome aufweisen, und wobei die gewellte Linie die Position kennzeichnet, über die die Linkergruppe an das organische Molekül der Formel 1 angebunden ist. In einer Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, insbesondere eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form - C(=O)O- auf.

Ausführungsformen der Wiederholungseinheiten sind Strukturen der Formeln 22 bis 27:

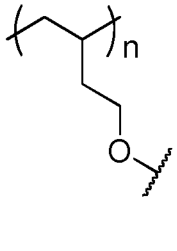
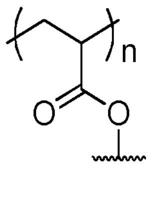
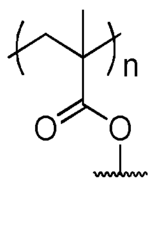
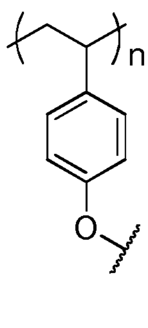
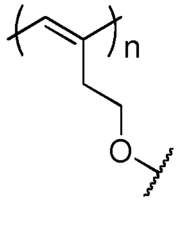
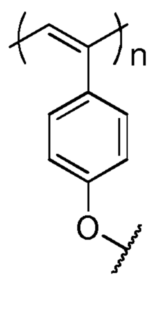

**Formel 22** **Formel 23** **Formel 24** **Formel 25** **Formel 26** **Formel 27**

Zur Herstellung der Polymere, die die Wiederholungseinheiten gemäß Formel 22 bis 27 aufweisen, werden die polymerisierbaren funktionellen Einheiten über eine Linkergruppe der Formeln 30 bis 35 die eine Hydroxyleinheit aufweisen, an das organische Molekül der Formel 1 angebunden und die daraus resultierenden Verbindungen mit sich selbst homopolymerisiert oder mit anderen geeigneten Monomeren copolymerisiert.

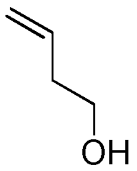
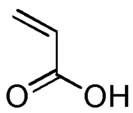
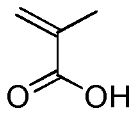
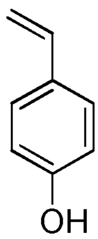
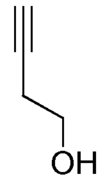
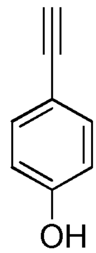

**Formel 30** **Formel 31** **Formel 32** **Formel 33** **Formel 34** **Formel 35**

Polymere, die eine Einheit gemäß Formel 20 oder Formel 21 aufweisen, können dabei entweder ausschließlich Wiederholungseinheiten mit einer Struktur der allgemeinen Formel 20 oder 21, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organische Moleküle, welche

- einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweisen und/oder
- eine Emissionslebensdauer von höchstens 50 $\mu$s aufweisen.

Eine weitere Ausführungsform der Erfindung betrifft die Verwendung eines Separators S in Form einer neutralen chemischen Einheit zur Bereitstellung eines erfindungsgemäßen organischen Moleküls, wobei mindestens eine erste chemische Einheit AF1, aufweisend ein konjugiertes System, insbesondere mindestens sechs in Konjugation stehende $\pi$-Elektronen und mindestens eine zweite chemische Einheit AF2, aufweisend ein konjugiertes System, insbesondere mindestens sechs in Konjugation stehende $\pi$-Elektronen, kovalent über den Separator S verknüpft. Vorteilhaft ist, wenn die Verknüpfung derart erfolgt, dass der Separator S eine direkte elektronische Wechselwirkung über konjugierte Bindungen zwischen dem konjugierten System der ersten chemischen Einheit AF1 und dem konjugierten System der zweiten chemischen Einheit AF2 unterbindet.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:

- organische lichtemittierende Dioden (OLEDs),
- Licht-emittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischem lichtemittierendem Bauelement (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischer lichtemittierender Diode, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des erfindungsgemäßen Bauelements wird das organische Molekül als Emissionsmaterial in einer Emissionsschicht eingesetzt wird, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform der Erfindung weist das optoelektronische Bauelement neben dem erfindungsgemäßen organischen Molekül mindestens ein Hostmaterial auf, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand (T) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform der Erfindung weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform der Erfindung weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform der Erfindung weist das optoelektronische Bauelement mindestens ein Hostmaterial aus einem Material gemäß Formel 1 auf.

In einer weiteren Ausführungsform des erfindungsgemäßen optoelektronischen Bauelements beinhaltet die lichtemittierende Schicht fluoreszente oder phosphoreszente Materialien, welche eine Struktur der Formel 1 aufweisen.

In einer weiteren Ausführungsform des erfindungsgemäßen optoelektronischen Bauelements bilden ein organisches Molekül gemäß Formel 1 und ein funktionelles Material, beispielsweise in Form eines weiteren Emitter-Materials, eines Host-Materials, oder eines weiteren organischen Moleküls, welches zur Bildung eines Exciplex mit dem Molekül gemäß Formel 1 befähigt ist, einen Exciplex. Funktionelle Materialien sind beispielsweise Hostmaterialien wie 1,3-Bis(N-carbazolyl)benzol (MCP), Elektronentransportmaterialien wie 2,2',2''-(1,3,5-Benzinetriyl)-tris(1-phenyl-1-H-benzimidazol) (TPBI) und Lochtransportmaterialien wie 4,4'-Bis(N-(1-naphthyl)-N-phenyl-amino)biphenyl (NPD) oder 4,4',4''-Tris[(*m*-methylphenyl) phenylamino]triphenylamin (MTDATA). Exciplexe sind Addukte aus elektronisch angeregten Molekülen und solchen im elektronischen Grundzustand, die zur Lichtemission fähig sind.

In einer weiteren Ausführungsform des erfindungsgemäßen optoelektronischen Bauelements ist die Emission durch thermisch aktivierte verzögerte Fluoreszenz (TADF) charakterisiert.

In einer weiteren Ausführungsform des erfindungsgemäßen optoelektronischen Bauelements werden organische Moleküle gemäß Formel 1 als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett ($T_1$)- und Singulett ($S_1$)-Energieniveaus des Hostmaterials energetisch höher liegen als die Triplett ($T_1$)- und Singulett ($S_1$)-Energieniveaus des organischen Moleküls, und wobei das organische Molekül Fluoreszenz oder thermisch aktivierte verzögerte Fluoreszenz (TADF) emittiert, und einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform ist das Verfahren gekennzeichnet durch die Verarbeitung des organischen Moleküls mittels eines Vakuumverdampfungsverfahrens oder aus einer Lösung.

In einer Ausführungsform der Erfindung ist das Verfahren gekennzeichnet durch das Aufbringen des organischen Moleküls auf einen Träger, wobei das Aufbringen insbesondere nass-chemisch, mittels kolloidaler Suspension oder mittels Sublimation erfolgt.

In einer weiteren Ausführungsform des erfindungsgemäßen Verfahrens wird mindestens eine Schicht

- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.

Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (Down-Konversion), insbesondere in einem optoelektronischen Bauelement der hier beschriebenen Art.

In einem weiteren Aspekt betrifft die Erfindung eine Anwendung, in der mindestens ein Material entsprechend Formel 1 oder Formel 2a oder Formel 2b, durch äußere energetische Anregung zum Leuchten angeregt wird. Die äußere Anregung kann elektronisch oder optisch oder radioaktiv sein.

## Beispiele

### Berechnungen nach der Dichtefuntionaltheorie

### Variante 1 (BP86)

Für die DFT-Rechnungen (Dichtefunktionaltheorie) wurde das BP86-Funktional (Becke, A. D. Phys. Rev. A1988, 38, 3098-3100; Perdew, J. P. Phys. Rev. B1986, 33, 8822-8827) und def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) verwendet. Zur numerischen Integration kam das m4-Grid zum Einsatz und die resolution-of-identity-Näherung (RI) (Häser, M.; Ahlrichs, R. J. Comput. Chem. 1989, 10, 104-111; Weigend, F.; Häser, M. Theor. Chem. Acc. 1997, 97, 331-340; Sierka, M.; Hogekamp, A.; Ahlrichs, R. J. Chem. Phys. 2003, 118, 9136-9148) wurde in allen Rechnungen verwendet. Die DFT-Rechnungen wurden mit dem Turbomole-Programmpaket (Version 6.5) (TURBOMOLE V6.4 2012, University of Karlsruhe/Forschungszentrum Karlsruhe GmbH, 1989-2007, TURBOMOLE GmbH, since 2007; http://www.turbomole.com) durchgeführt.

### Variante 2 (TD-B3LYP)

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F.; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem.

Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, University of Karlsruhe und Forschungszentrum Karlsruhe GmbH, 1989-2007, TURBOMOLE GmbH, since 2007; http://www. turbomole.com) durchgeführt.

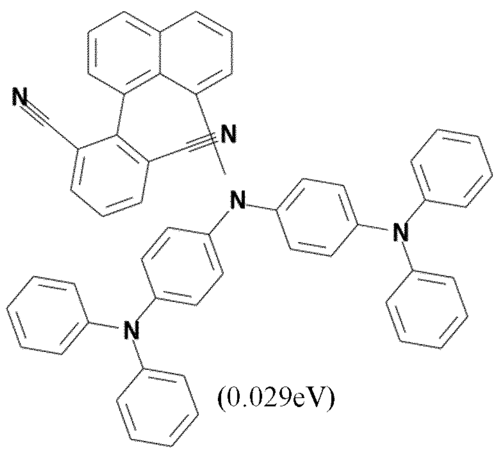

(0.029eV)

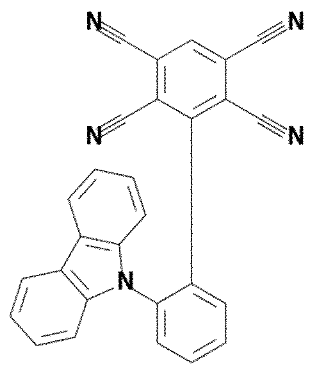

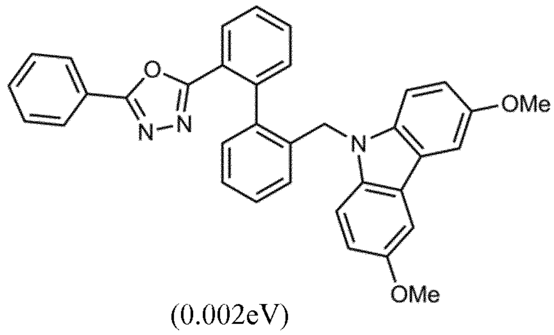

(0.002eV)

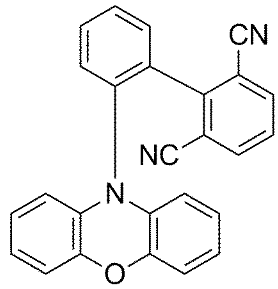

(0.085eV) (0.046eV)

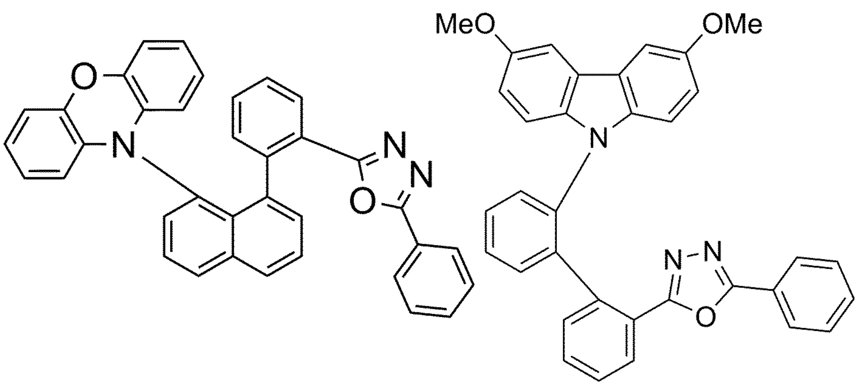

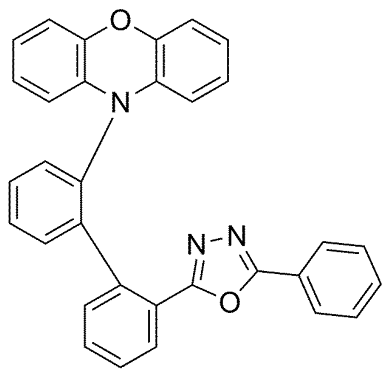

(0.089eV) (0.024eV) (0.017eV)

## Figuren

Es zeigen

Figur 1: Schematische Darstellung des Aufbaus einer organischen lichtemittierenden Diode (OLED).

Figur 2: Schematische Darstellung des Energieniveaudiagrammes (relative Energie in eV) eines erfindungsgemäßen Emittermoleküls, aufweisend einen Separator (Lichtemission resultiert aus dem Übergang LUMO AF1 hin zu HOMO AF2).

**Patentansprüche**

**1.** Organisches Molekül, aufweisend eine Struktur der Formel 1

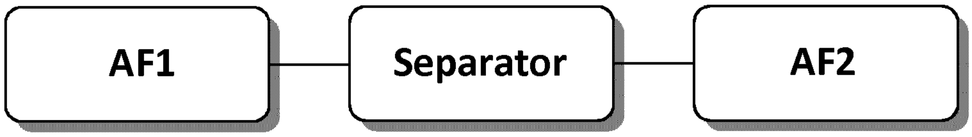


**Formel 1**

wobei gilt:

AF1: eine erste chemische Einheit, aufweisend ein konjugiertes System;
AF2: eine zweite chemische Einheit, aufweisend ein konjugiertes System, mit AF1 ≠ AF2;
Separator ist eine chemische Einheit, die AF1 und AF2 verknüpft;
wobei der Separator eine Struktur der Formel 2 aufweist

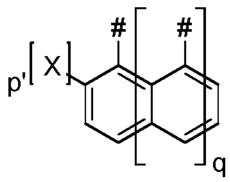

**Formel 2**

wobei die Anknüpfungspositionen für die chemischen Einheiten AF1 und AF2 durch den Platzhalter # gekennzeichnet sind,
X = #, $CH_2$#, oder

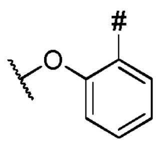

p' = 0 oder 1;
q = 0 oder 1;
wobei p' ≠ q;
wobei die chemische Einheit AF1 ausgewählt ist aus der Gruppe der folgenden Strukturen, die optional mindestens einen Substituenten aufweisen:

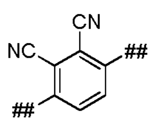

153

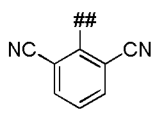

150

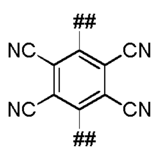

150-1

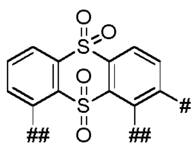

46

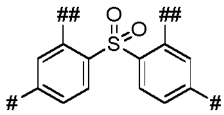

111

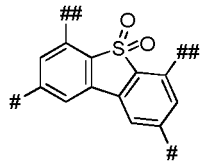

198

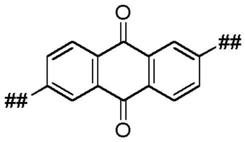

36

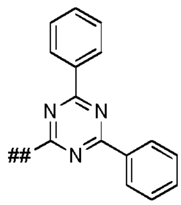

233

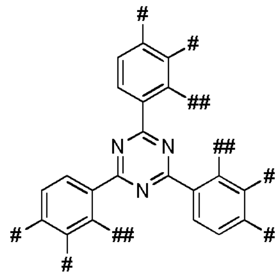

233-1

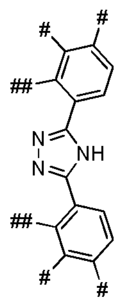

222

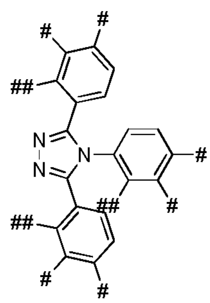

222-1

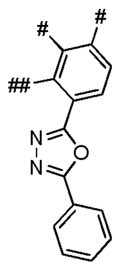

118

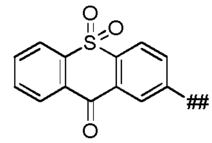

43

wobei gilt

#, ##: mögliche Anknüpfungspositionen an den Separator;
und wobei die chemische Einheit AF2 unabhängig voneinander ausgewählt ist aus der Gruppe der folgenden Strukturen, die optional mindestens einen Substituenten aufweisen:

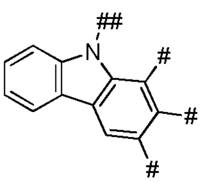

328

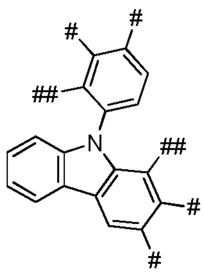

55

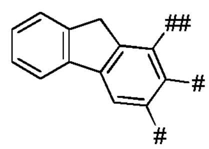

106

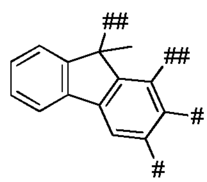

106-1

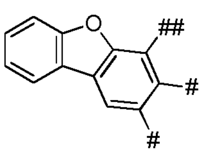

432

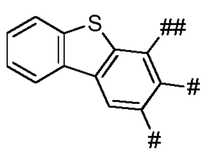

433

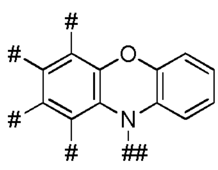

41

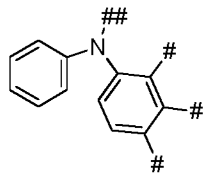
223

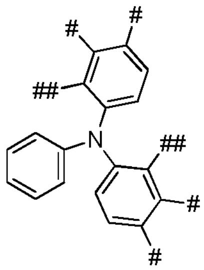
317

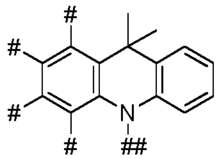
337

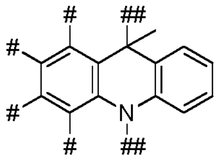
337-1

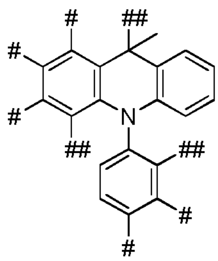
337-2

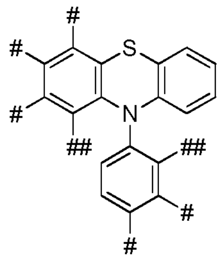
77

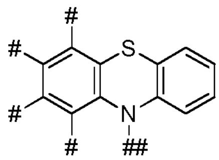
77-1

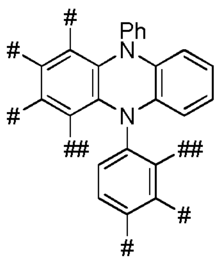
425

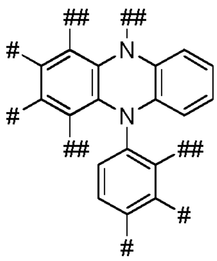
425-1

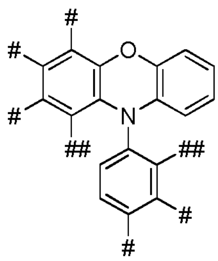
78

und wobei gilt:

#, ##: mögliche Anknüpfungspositionen an den Separator.

**2.** Organisches Molekül nach Anspruch 1, wobei der Separator ausgewählt ist aus der Gruppe der folgenden Strukturen

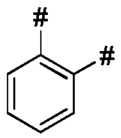
S-a

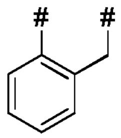
S-b

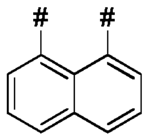
S-c

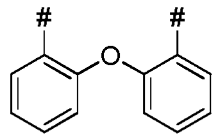
S-e

wobei # die Positionen kennzeichnet, an denen die chemischen Einheiten AF1 und AF2 an den Separator gebunden sind;

**3.** Organisches Molekül nach Anspruch 1 oder 2, aufweisend eine Struktur der Formel 3, wobei die Anknüpfung der chemischen Einheit AF1 an den Separator über die Anknüpfungsposition ## der chemischen Einheit AF1 erfolgt, die an einem C-Atom lokalisiert ist;
wobei die Anknüpfung der chemischen Einheit AF2 an den Separator optional über die Anknüpfungsposition ## oder über ein Stickstoffatom erfolgt;
wobei die chemische Einheit AF2 ausgewählt ist aus der Gruppe bestehend aus 328, 41, 223, 77-1, 337-1 und

425-1, wenn die Anknüpfungsposition an einem Strickstoffatom lokalisiert ist:

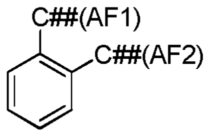

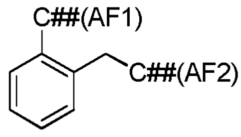

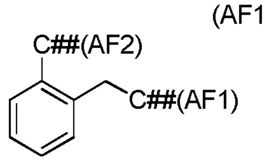

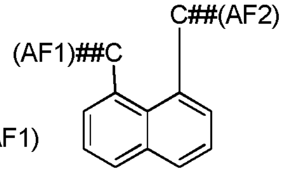

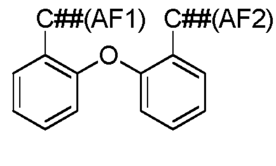

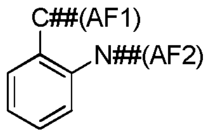

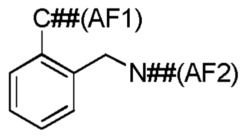

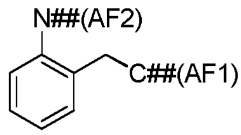

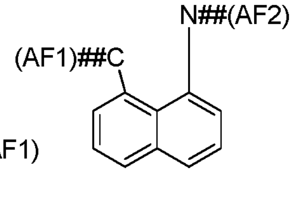

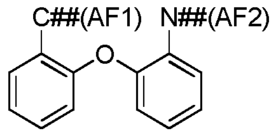

**Formel 3**

wobei die Anknüpfung der chemischen Einheit AF2 insbesondere über ein Stickstoffatom N## oder über ein Kohlenstoffatom C## erfolgt.

**4.** Organisches Molekül nach einem oder mehreren der Ansprüche 1 bis 3, wobei das organische Molekül an mindestens einer chemisch substituierbaren Position mindestens einen weiteren Rest R, insbesondere zur Steigerung der Löslichkeit und/oder der Polymerisierbarkeit, aufweist, wobei
R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein mono- oder polycyclisches, aliphatisches, aromatisches und/oder benzoannelliertes Ringsystem bilden;
$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $AS(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das

jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, $-C\equiv C-$, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, $-C\equiv C-$, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, $-C\equiv C-$, oder eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl,

$N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C≡C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-,$-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

und wobei für polymerisierbare Reste gilt:

polymerisierbare Reste sind solche Reste, die polymerisierbare funktionelle Einheiten tragen, die mit sich selbst homopolymerisiert oder mit anderen Monomeren copolymiersiert werden können, wodurch das organische Molekül als Polymer mit Wiederholungseinheiten nach den Formeln 20 und/oder 21 erhalten werden

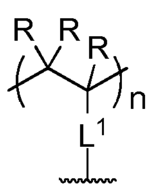

**Formel 20**

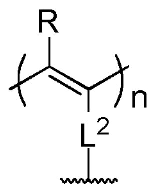

**Formel 21**

mit

gewellte Linie = Position, über die die Linkergruppe $L^1$ oder $L^2$ an das organische Molekül gebunden ist;

$L^1$ und $L^2$ = gleiche oder verschiedene Linkergruppen, aufweisend zwischen 0 bis 20 Kohlenstoffatome; oder aufweisend eine Form -C(=O)O;

und wobei insbesondere $L^1$ und $L^2$ = $-X-L^3$ mit X = O oder S;

$L^3$ = eine weitere Linkergruppe ausgewählt aus der Gruppe aus einersubstituierten und unsubstituierten Alkylengruppe (linear, verzweigt oder cyclisch) und einer substituierten und unsubstituierten Arylengruppe, wobei

auch Kombinationen möglich sind.

5. Verwendung eines organischen Moleküls nach Anspruch 1 bis 4 als lumineszierender Emitter und/oder als Hostmaterial und/oder als Elektronentransportmaterial und/oder als Lochinjektionsmaterial und/oder als Lochblockiermaterial in einem optoelektronischen Bauelement.

6. Verwendung nach Anspruch-5, wobei das optoelektronische Bauelement ausgewählt ist aus der Gruppe bestehend aus:

    • organischen lichtemittierenden Dioden (OLEDs),
    • Licht-emittierenden elektrochemischen Zellen,
    • OLED-Sensoren, insbesondere in nicht hermetisch nach außen abgeschirmten Gas- und Dampf-Sensoren,
    • organischen Dioden
    • organischen Solarzellen,
    • organischen Transistoren,
    • organischen Feldeffekttransistoren,
    • organischen Lasern und
    • Down-Konversions-Elementen.

7. Verwendung nach Anspruch 5 oder 6, wobei die Konzentration des organischen Moleküls als Emitter in optischen Licht emittierenden Bauelementen, insbesondere in OLEDs, zwischen 5 Gew. % und 80 Gew. % beträgt.

8. Optoelektronisches Bauelement, aufweisend ein organisches Molekül nach Anspruch 1 bis 4, insbesondere ausgeformt als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

9. Optoelektronisches Bauelement nach Anspruch 8, aufweisend

    - ein Substrat,
    - eine Anode und
    - eine Kathode, wobei die Anode und die Kathode auf das Substrat aufgebracht sind, und
    - mindestens eine lichtemittierende Schicht, die zwischen Anode und Kathode angeordnet ist und die ein organisches Molekül nach Anspruch 1 bis 4 enthält.

10. Optoelektronisches Bauelement nach Anspruch 8 oder 9, in dem die lichtemittierende Schicht mindestens ein Hostmaterial aufweist, wobei der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls nach Anspruch 1 bis 4.

11. Optoelektronisches Bauelement nach Anspruch 8 bis 10, aufweisend mindestens ein Hostmaterial bestehend aus einem Material gemäß Anspruch 1 bis 4.

12. Optoelektronisches Bauelement nach Anspruch 8 bis 11, wobei die lichtemittierende Schicht fluoreszente oder phosphoreszente Materialien beinhaltet und wobei es sich bei den Materialien der lichtemittierenden Schicht um organische Moleküle nach Anspruch 1 bis 4 handelt.

13. Optoelektronisches Bauelement nach Anspruch 12, in dem ein organisches Molekül gemäß Anspruch 1 bis 4 zusammen mit einem funktionellen Material einen Exciplex bilden.

14. Optoelektronisches Bauelement nach Anspruch 8 bis 13, in dem ein organisches Molekül nach Anspruch 1 bis 4 als Ladungstransportschicht verwendet wird.

15. Verfahren zur Herstellung eines optoelektronischen Bauelements, wobei ein organisches Molekül nach Anspruch 1 bis 4 verwendet wird, wobei die die Verarbeitung des organischen Moleküls insbesondere mittels eines Vakuumverdampfungsverfahrens oder aus einer Lösung erfolgt.

**Claims**

**1.** Organic molecule, comprising a structure of formula 1

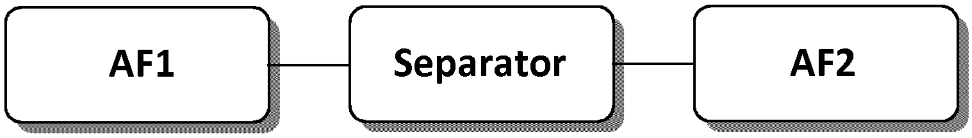


**Formula 1**

where:

AF1: a first chemical unit, comprising a conjugated system;
AF2: a second chemical unit, comprising a conjugated system, with AF1 ≠ AF2;
separator is a chemical unit, which connects AF1 and AF2;
where the separator comprises a structure of formula 2

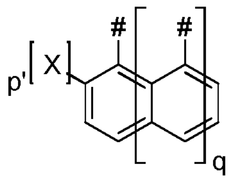

**Formula 2**

where the attachment position for the chemical units AF1 and AF2 is **characterized by** the placeholder #,
X = #, $CH_2$#, or

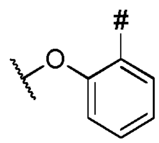

p' = 0 or 1;
q = 0 or 1;
where p' ≠ q;
where the chemical unit AF1 is selected from the group of the following structures, which comprise optionally at least one substituent:

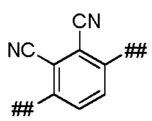

153

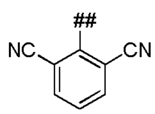

150

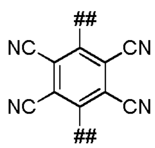

150-1

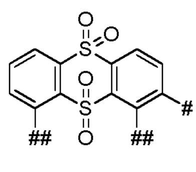

46

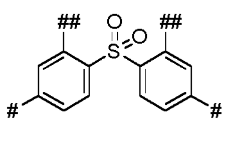

111

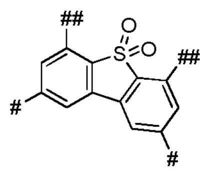

198

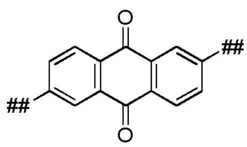

36

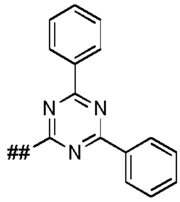

233

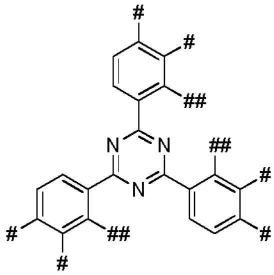

233-1

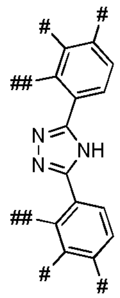

222

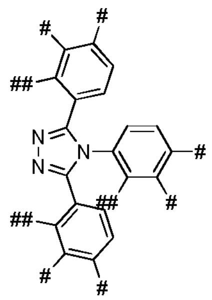

222-1

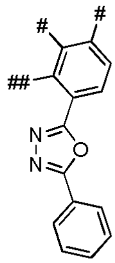

118

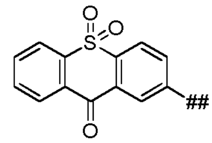

43

where

#, ##: possible attachment positions to the separator;
and where the chemical unit AF2 independently of each other is selected from the group of the following structures, which optionally comprise at least one substituent:

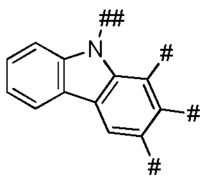

328

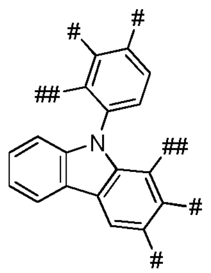

55

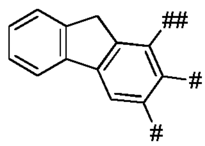

106

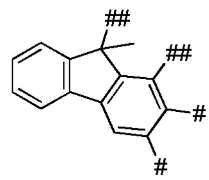

106-1

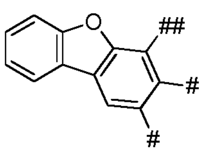

432

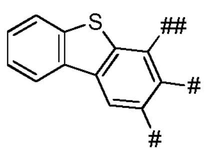

433

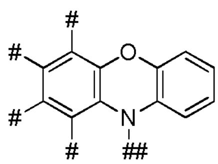

41

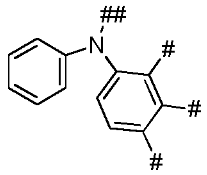

223

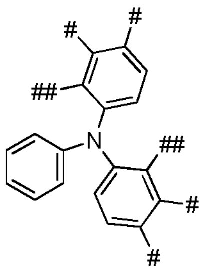

317

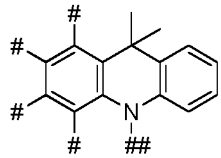

337

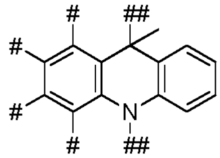

337-1

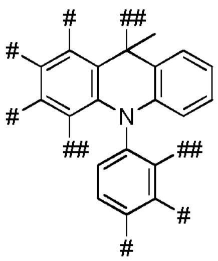

337-2

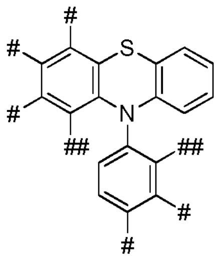

77

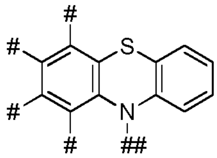

77-1

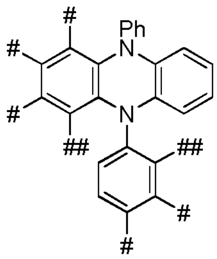

425

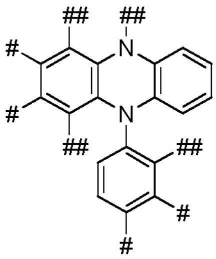

425-1

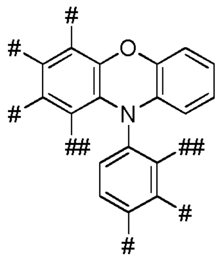

78

and where:

#, ##: possible attachment positions to the separator.

**2.** Organic molecule according to claim 1, wherein the separator is selected from the group of the following structures

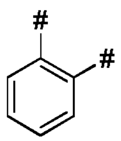

S-a

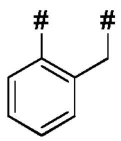

S-b

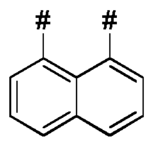

S-c

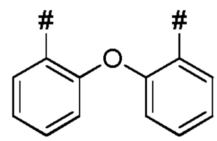

S-e

where # characterizes the positions at which the chemical units AF1 and AF2 are bound to the separator.

**3.** Organic molecule according to claim 1 or 2, comprising a structure of formula 3, wherein the attachment of the chemical unit AF1 to the separator is via the attachment position ## of the chemical unit AF1, which is located at a carbon atom;
where the attachment of the chemical unit AF2 to the separator is optionally via the attachment position ## or is via a nitrogen atom;
where the chemical unit AF2 is selected from the group consisting of 328, 41, 223, 77-1, 337-1 and 425-1, if the attachment position is located at a nitrogen atom:

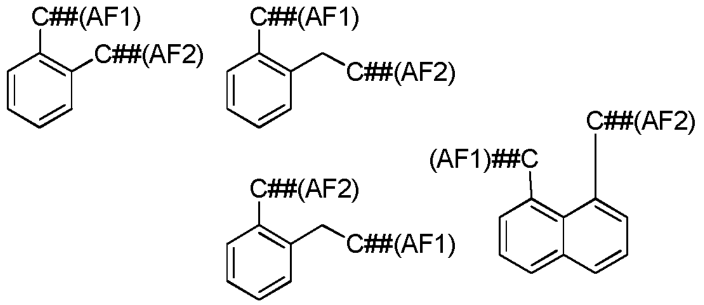

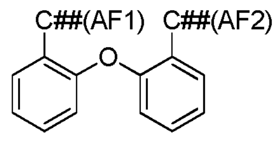

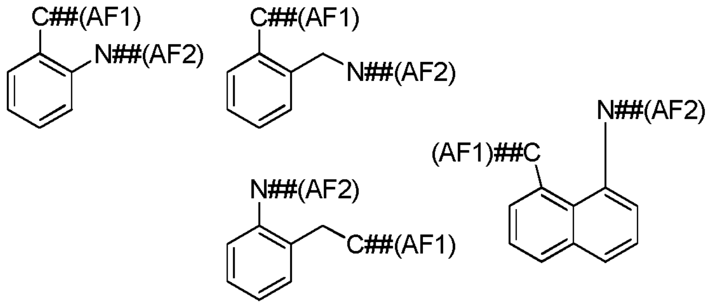

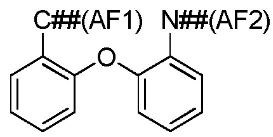

**Formula 3**

where the attachment of the chemical unit AF2 is in particular via a nitrogen atom N## or is via a carbon atom C##.

**4.** Organic molecule according to one ore more of claims 1 to 3, wherein the organic molecule comprises at at least one chemically substitutable position at at least one further moiety R, in particular to increase the solubility and/or polymerizability, wherein

R is at each instance independently of each other selected from the group consisting of H, deuterium, phenyl, naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^2$ moieties, or an aryloxy or heteroaryloxy group which has 5 to 60 aromatic ring atoms and may be substituted by one or more $R^9$ moieties, or a diarylamino group, diheteroarylamino group or arylheteroarylamino group which has 10 to 40 aromatic ring atoms and may be substituted by one or more $R^9$ moieties, or a combination of these systems; at the same time, two or more of these R substituents together may also form a mono- or polycyclic, aliphatic, aromatic and/or benzofused ring system;

$R^2$ is at each instance independently of each other selected from the group consisting of H, deuterium, phenyl, naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$, $P(=S)(R^7)_2$, $As(=S)(R^7)_2$, $S(=O)R^3$, $S(=O)_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ moieties, or an aryloxy or heteroaryloxy group which has 5 to 60 aromatic ring atoms and may be substituted by one or more $R^9$ moieties, or a diarylamino group, diheteroarylamino group or arylheteroarylamino group which has 10 to 40 aromatic ring atoms and may be substituted by one or more $R^9$ moieties, or a combination of these systems; at the same time, two or more of these $R^2$ substituents together may also form a mono- or polycyclic, aliphatic, aromatic and/or benzofused ring system;

$R^3$ is at each instance independently of each other selected from the group consisting of H, deuterium, phenyl, naphthyl, $CF_3$ or an aliphatic, aromatic and/or heteroaromatic hydrocarbyl moiety which has 1 to 20 carbon atoms and in which one or more hydrogen atoms may also be replaced by F or $CF_3$; at the same time, two or more $R^3$ substituents together may also form a mono- or polycyclic aliphatic ring system;

$R^4$ is at each instance independently of each other selected from the group consisting of H, deuterium, phenyl, naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$,-O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^8$ moieties, or an aryloxy or heteroaryloxy group which has 5 to 60 aromatic ring atoms and may be substituted by one or more $R^9$ moieties, or a diarylamino group, diheteroarylamino group or arylheteroarylamino group which has 10 to 40 aromatic ring atoms and may be substituted by one or more $R^9$ moieties, or a combination of these systems; at the same time, two or more of these $R^4$ substituents together may also form a mono- or polycyclic, aliphatic, aromatic and/or benzofused ring system;

$R^5$ is at each instance independently of each other selected from the group consisting of phenyl, naphthyl, $CF_3$, $C(=O)R^3$, $P(=O)(R^7)_2$, $As(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ moieties, or an aryloxy or heteroaryloxy group which has 5 to 60 aromatic ring atoms and may be substituted by one or more $R^9$ moieties, or a diarylamino group, diheteroarylamino group or arylheteroarylamino group which has 10 to 40 aromatic ring atoms and may be substituted by one or more $R^9$ moieties, or a combination of these systems; at the same time, two or more of these $R^5$ substituents together may also form a mono- or polycyclic, aliphatic, aromatic and/or benzofused ring system;

$R^6$ is at each instance independently of each other selected from the group consisting of phenyl, naphthyl, $CF_3$, $Si(R^4)_3$, $C(=O)R^3$, $P(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)$, $-As(=S)(R^7)$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ moieties, or an aryloxy or heteroaryloxy group which has 5 to 60 aromatic ring atoms and may be substituted by one or more R* moieties, or a diarylamino group, diheteroarylamino group or arylheteroarylamino group which has 10 to 40 aromatic ring atoms and may be substituted by one or more $R^9$ moieties, or a combination of these systems; at the same time, two or more of these $R^6$ substituents together may also form a mono- or polycyclic, aliphatic, aromatic and/or benzofused ring system;

$R^7$ is at each instance independently of each other selected from the group consisting of phenyl, naphthyl, $N(R^2)_2$, CN, $CF_3$, $C(=O)OR^3$, $C(=O)N(R^3)_2$, $Si(R^4)_3$, $C(=O)R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ moieties, where one or more adjacent $CH_2$ groups may be replaced by $-R^9C=CR^9-$, -C≡C-, or an adjacent $CH_2$ group may be replaced by $-Si(R^4)_2-$, $-Ge(R^4)_2-$, $-Sn(R^4)_2$, -C(=O)-, -C(=S)-,-C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ moieties, or an aryloxy or heteroaryloxy group which has 5 to 60 aromatic ring atoms and may be substituted by one or more $R^9$ moieties, or a diarylamino group, diheteroarylamino group or arylheteroarylamino group which has 10 to 40 aromatic ring atoms and may be substituted by one or more $R^3$ moieties, or a combination of these systems; at the

same time, two or more of these $R^7$ substituents together may also form a mono- or polycyclic, aliphatic, aromatic and/or benzofused ring system;
$R^8$ is at each instance independently of each other selected from the group consisting of H, deuterium, phenyl, naphthyl, F, $CF_3$ or an aliphatic, aromatic and/or heteroaromatic hydrocarbyl moiety which has 1 to 20 carbon atoms and in which one or more hydrogen atoms may also be replaced by F or $CF_3$; at the same time, two or more $R^8$ substituents together may also form a mono- or polycyclic aliphatic ring system;
$R^9$ is at each instance independently of each other selected from the group consisting of H, deuterium, phenyl, naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^8$ moieties, where one or more adjacent $CH_2$ groups may be replaced by $-R^3C=CR^3-$, -C≡C-, or an adjacent $CH_2$ group may be replaced by $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^8$ moieties, or an aryloxy or heteroaryloxy group which has 5 to 60 aromatic ring atoms and may be substituted by one or more $R^3$ moieties, or a diarylamino group, diheteroarylamino group or arylheteroarylamino group which has 10 to 40 aromatic ring atoms and may be substituted by one or more $R^8$ moieties, or a combination of these systems; at the same time, two or more of these $R^9$ substituents together may also form a mono- or polycyclic, aliphatic, aromatic and/or benzofused ring system;
and where for polymerizable moieties:
polymerizable moieties are moieties that bear functional units that can be homopolymerized with themselves or copolymerized with other monomers, whereby the organic molecule is obtained as polymer having repeatable units according to the formulae 20 and/or 21

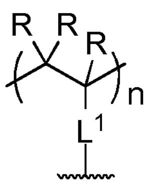

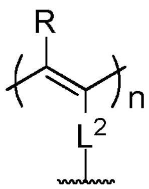

**Formula 20** **Formula 21**

with

wavy line = position via which the linker group $L^1$ or $L^2$ is bound to the organic molecule; $L^1$ and $L^2$ = identical or different linker groups having from 0 to 20 carbon atoms; or
comprising a -C(=O)O form;
and where, in particular, $L^1$ and $L^2$ = $-X-L^3$ with X = O or S;
$L^3$ = a further linker group selected from the group of a substituted and unsubstituted alkylene group (linear, branched or cyclic) and a substituted and unsubstituted arylene group, where combinations are also possible.

5. Use of an organic molecule according to claims 1 to 4 as luminescent emitter and/or as host material and/or as electron transport material and/or as hole injection material and/or as hole blocking material in an optoelectronic component.

6. Use according to claim 5, wherein the optoelectronic component is selected from the group consisting of:

- organic light-emitting diodes (OLEDs),
- light-emitting electrochemical cells,
- OLED sensors, especially in gas and vapor sensors that are not hermetically shielded from the outside,
- organic diodes,
- organic solar cells,
- organic transistors,
- organic field-effect transistors,

- organic lasers and
- down-conversion elements.

**7.** Use according to claim 5 or 6, wherein the concentration of the organic molecule as emitter in optical light-emitting components, in particular in OLEDs, is between 5 weight% and 80 weight%.

**8.** Optoelectronic component comprising an organic molecule according to claims 1 to 4, in particular in the form of a component selected from the group consisting of organic light-emitting diode (OLED), light-emitting electrochemical cell, OLED sensor, especially in gas and vapor sensors that are not hermetically shielded from the outside, organic diode, organic solar cell, organic transistor, organic field-effect transistor, organic laser and down-conversion element.

**9.** Optoelectronic component according to claim 8, comprising

- a substrate,
- an anode and
- a cathode, where the anode and the cathode have been applied to the substrate, and
- at least one light-emitting layer which is disposed between the anode and the cathode and which consists of an organic molecule according to claims 1 to 4.

**10.** Optoelectronic component according to claim 8 or 9, in which the light-emitting layer comprises at least one host material, where the excited singlet state ($S_1$) and/or the excited triplet state ($T_1$) of the at least one host material is higher than the excited singlet state ($S_1$) and/or the excited triplet state ($T_1$) of the organic molecule according to claims 1 to 4.

**11.** Optoelectronic component according to claims 8 to 10, comprising at least one host material consisting of a material according to claims 1 to 4.

**12.** Optoelectronic component according to claims 8 to 11, wherein the light-emitting layer comprises fluorescent or phosphorescent materials and wherein the materials in the light-emitting layer are organic molecules according to claims 1 to 4.

**13.** Optoelectronic component according to claim 12, in which an organic molecule according to claims 1 to 4 forms an exciplex together with a functional material.

**14.** Optoelectronic component according to claims 8 to 13, in which an organic molecule according to claim 1 to 4 is used a charge transport layer.

**15.** Method for producing an optoelectronic component, wherein an organic molecule according to claims 1 to 4 is used, wherein the processing of the organic molecule is carried out in particular via a vacuum evaporation process or from a solution.

## Revendications

**1.** Molécule organique, comportant une structure de formule 1

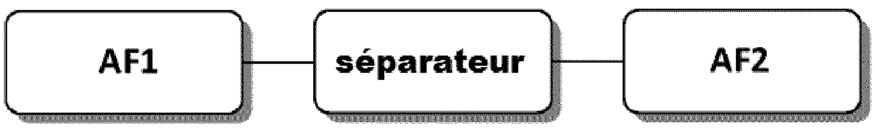


**Formule 1**

dans laquelle :

AF1 : une première unité chimique, comportant un système conjugué ;
AF2 : une deuxième unité chimique, comportant un système conjugué, avec AF1 ≠ AF2 ;

séparateur est une unité chimique qui relie AF1 et AF2 ;
le séparateur présentant une structure de formule 2

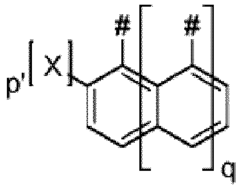

**Formule 2**

dans laquelle les positions d'attachement des unités chimiques AF1 et AF2 sont **caractérisées par** les symboles #
X = #, $CH_2$#, ou

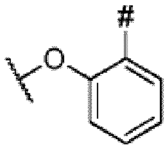

p' = 0 ou 1 ;
q = 0 ou 1 ;
p' étant ≠ q ;
l'unité chimique AF1 étant choisie dans le groupe des structures suivantes, qui en option comportent au moins un substituant :

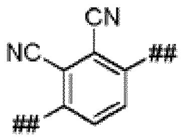

153

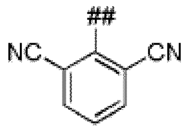

150

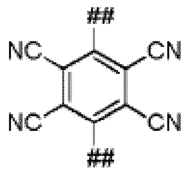

150-1

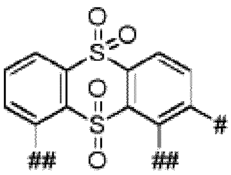

46

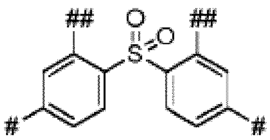

111

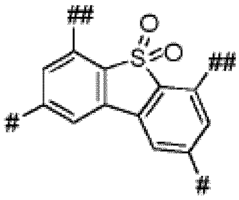

198

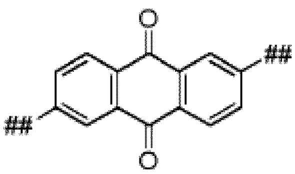

36

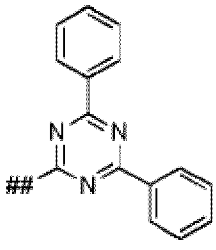

233

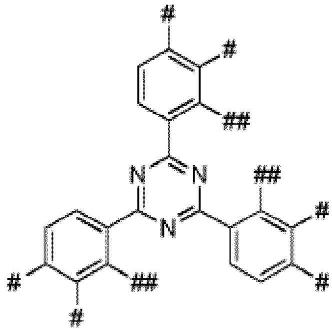

233-1

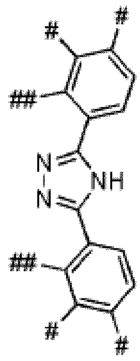
222

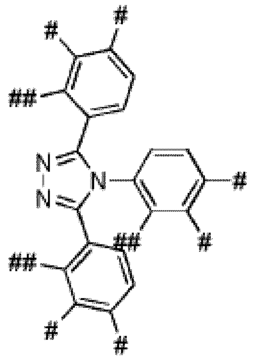
222-1

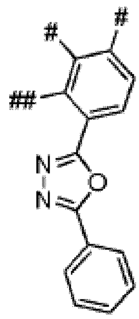
118

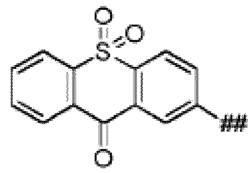
43

où il s'applique que

#, ## : positions d'attachement possibles au séparateur ;
et où l'unité chimique AF2 est choisie chaque fois indépendamment dans le groupe des structures suivantes, qui en option comportent au moins un substituant :

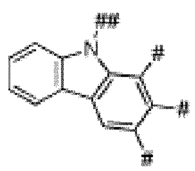
328

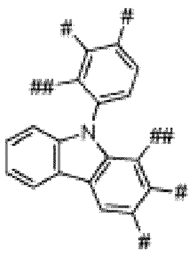
55

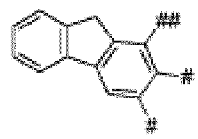
106

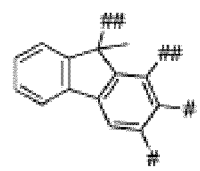
106-1

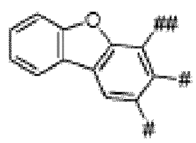
432

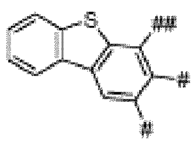
433

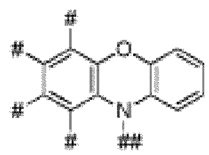
41

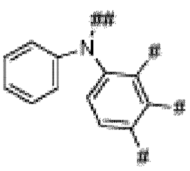
223

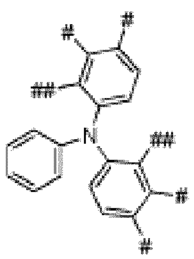
317

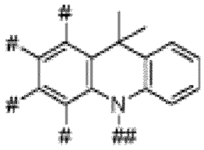
337

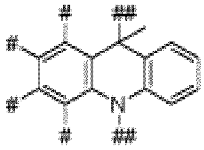
337-1

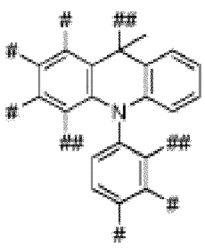

337-2

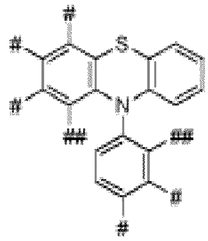

77

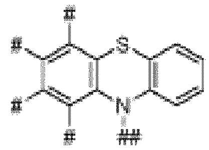

77-1

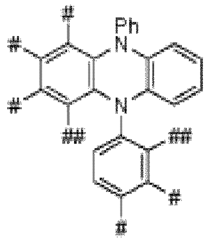

425

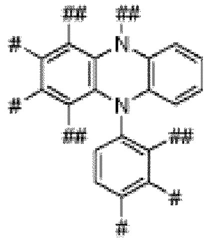

425-1

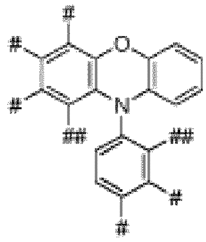

78

et où il s’applique que

#, ## : positions d’attachement possibles au séparateur.

2. Molécule organique selon la revendication 1, dans laquelle le séparateur est choisi dans le groupe des structures suivantes :

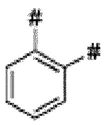

S-a

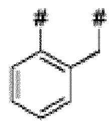

S-b

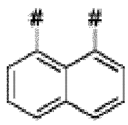

S-c

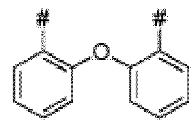

S-e

où # caractérise les positions au niveau desquelles les unités chimiques AF1 et AF2 sont liés au séparateur.

3. Molécule organique selon la revendication 1 ou 2, présentant une structure de formule 3, dans laquelle l’attachement de l’unité chimique AF1 au séparateur s’effectue par la position d’attachement ## de l’unité chimique AF1 qui est localisée au niveau d’un atome de carbone ;
dans laquelle l’attachement de l’unité chimique AF2 au séparateur s’effectue en option par la position d’attachement ## ou par un atome d’azote ;
dans laquelle l’unité chimique AF2 est choisie dans le groupe constitué par 328, 41, 223, 77-1, 337-1 et 421-1, lorsque la position d’attachement est localisée au niveau d’un atome d’azote :

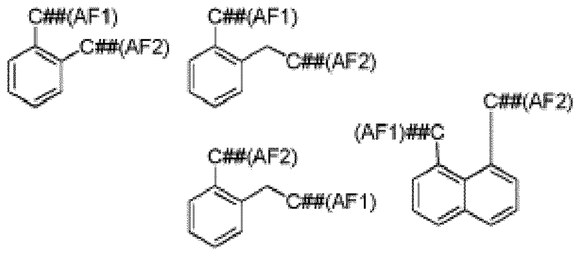

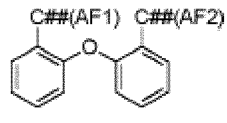

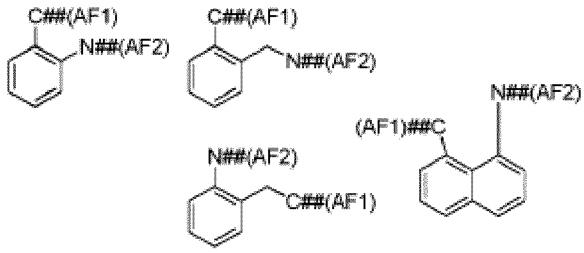

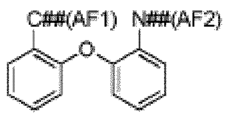

**Formule 3**

l'attachement de l'unité chimique AF2 s'effectuant en particulier par un atome d'azote N## ou par un atome de carbone C##.

4. Molécule organique selon une ou plusieurs des revendications 1 à 3, où la molécule organique comporte en au moins une position apte à la substitution chimique au moins un autre radical R, en particulier pour l'augmentation de la solubilité et/ou de l'aptitude à la polymérisation, où
R est choisi indépendamment à chaque occurrence dans le groupe constitué par H, le deutérium, les groupes phényle, naphtyle, F, Cl, Br, I, $N(R^2)_2$, -CN, -NC, -SCN, $-CF_3$, $-NO_2$,-OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)P^3$, $P(=O)(R^7)_2$, $As(=O)(R^7)_2$, $P(=S)(R^7)_2$, $As(=S)(R^7)_2$, $S(=O)R^3$, $S{=}NR^3$, $S(=O)NR^3$, $S(=O)_2NR^3$, $S(=O)_2R^3$, $O-S(=O)_2R^3$, $SF_5$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par$-R^9C{=}CR^9-$, -C°C-, ou un groupe $CH_2$ voisin pouvant être remplacé par $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^2$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants R peuvent en outre également former ensemble un système cyclique mono- ou polycyclique, aliphatique, aromatique et/ou soudé à un noyau benzénique ;
$R^2$ est choisi indépendamment à chaque occurrence dans le groupe constitué par H, le deutérium, les groupes phényle, naphtyle, $-CF_3$, $C(=O)OR^3$, $C(=O)N(R^3)_2$, $Si(R^4)_3$, $C(=O)R^3$ $P(=O)(R^7)_2$, $As(=O)(R^7)_2$, $P(=S)(R^7)_2$, $As(=S)(R^7)_2$, $S(=O)R^3$, $S(=O)_2R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C{=}CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de

10 à 40 atomes formant le cycle aromatique, qui peut être substitué par un ou plusieurs radicaux $R^9$, ou une combinaison de ces systèmes ; deux ou plus de deux de ces substituants $R^2$ peuvent en outre également former ensemble un système cyclique mono- ou polycyclique, aliphatique, aromatique et/ou soudé à un noyau benzénique ;

$R^3$ est choisi indépendamment à chaque occurrence dans le groupe constitué par H, le deutérium, les groupes phényle, naphtyle, $CF_3$ ou un radical hydrocarboné aliphatique, aromatique et/ou hétéroaromatique ayant de 1 à 20 atomes de carbone, dans lequel un ou plusieurs atomes d'hydrogène peuvent également être remplacés par F ou $CF_3$; deux ou plus de deux de ces substituants $R^3$ peuvent en outre également former ensemble un système cyclique mono- ou polycyclique, aliphatique ;

$R^4$ est choisi indépendamment à chaque occurrence dans le groupe constitué par H, le deutérium, les groupes phényle, naphtyle, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^8$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^4$ peuvent en outre également former ensemble un système cyclique mono- ou polycyclique, aliphatique, aromatique et/ou soudé à un noyau benzénique ;

$R^5$ est choisi indépendamment à chaque occurrence dans le groupe constitué par les groupes phényle, naphtyle, $CF_3$, $C(=O)R^3$, $P(=O)(R^7)_2$, $As(=O)(R^7)_2$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^5$ peuvent en outre également former ensemble un système cyclique mono- ou polycyclique, aliphatique, aromatique et/ou soudé à un noyau benzénique ;

$R^6$ est choisi indépendamment à chaque occurrence dans le groupe constitué par les groupes phényle, naphtyle, $CF_3$, $Si(R^4)_3$, $C(=O)R^3$, $P(=O)(R^7)_2$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux R*, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^6$ peuvent en outre également former ensemble un système cyclique mono- ou polycyclique, aliphatique, aromatique et/ou soudé à un noyau benzénique ;

$R^7$ est choisi indépendamment à chaque occurrence dans le groupe constitué par les groupes phényle, naphtyle, $N(R^2)_2$, CN, $CF_3$, $C(=O)OR^3$, $C(=O)N(R^3)_2$, $Si(R^4)_3$, $C(=O)R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui

peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^3$, ou une combinaison de ces systèmes ; deux ou plus de deux de ces substituants $R^7$ peuvent en outre également former ensemble un système cyclique mono- ou polycyclique, aliphatique, aromatique et/ou soudé à un noyau benzénique ;

$R^8$ est choisi indépendamment à chaque occurrence dans le groupe constitué par H, le deutérium, les groupes phényle, naphtyle, F, $CF_3$, ou un radical hydrocarboné aliphatique, aromatique et/ou hétéroaromatique ayant de 1 à 20 atomes de carbone, dans lequel un ou plusieurs atomes d'hydrogène peuvent également être remplacés par F ou $CF_3$; deux ou plus de deux de ces substituants $R^8$ peuvent en outre également former ensemble un système cyclique mono- ou polycyclique, aliphatique ;

$R^9$ est choisi indépendamment à chaque occurrence dans le groupe constitué par H, le deutérium, les groupes phényle, naphtyle, $N(R^2)_2$, CN, $CF_3$, $NO_2$, OH, COOH, $C(=O)OR^3$ $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plu6sieurs radicaux $R^8$, un ou plusieurs groupes $CH_2$ non contigus pouvant être remplacés par $-R^3C=CR^3-$, -C°C-, ou un groupe $CH_2$ voisin pouvant être remplacé par $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^8$, ou un groupe aryloxy ou hétéroaryloxy ayant de 5 à 60 atomes formant le cycle aromatique, qui peut être substitué par un ou plusieurs radicaux $R^3$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^8$, ou une combinaison de ces systèmes ; deux ou plus de deux de ces substituants $R^9$ peuvent en outre également former ensemble un système cyclique mono- ou polycyclique, aliphatique, aromatique et/ou soudé à un noyau benzénique ;

et où pour les radicaux aptes à la polymérisation il s'applique que :

les radicaux aptes à la polymérisation sont des radicaux qui portent des unités fonctionnelles aptes à la polymérisation, qui peuvent être homopolymérisés avec eux-mêmes ou copolymérisés avec d'autres monomères, de sorte qu'on obtient la molécule organique sous forme de polymère à motifs répétitifs selon la formule 20 et/ou la formule 21

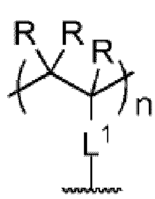 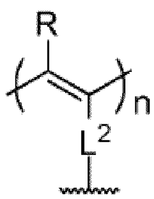

**Formule 20    Formule 21**

où

trait ondulé = position par laquelle le groupe de liaison $L^1$ ou $L^2$ est attaché à la molécule organique ;

$L^1$ et $L^2$ = des groupes de liaison identiques ou différents, comportant entre 0 et 20 atomes de carbone ; ou présentant une forme -C(C=O)O ;

et où en particulier $L^1$ et $L^2$ = $-X-L^3$ où X = O ou S ;

$L^3$ = un autre groupe de liaison choisi dans le groupe constitué par un groupe alkylène substitué et un groupe alkylène non substitué (linéaires, ramifiés ou cycliques) et un groupe arylène substitué et un groupe arylène non substitué, des associations étant également possibles.

5. Utilisation d'une molécule organique selon l'une quelconque des revendications 1 à 4 en tant qu'émetteur luminescent et/ou en tant que matériau hôte et/ou en tant que matériau transporteur d'électrons et/ou en tant que matériau d'injection de trous et/ou en tant que matériau de blocage de trous dans un composant optoélectronique.

6. Utilisation selon la revendication 5, dans laquelle le composant optoélectronique est choisi dans le groupe constitué par :

• des diodes électroluminescentes organiques (OLED),
• des cellules électrochimiques photoémettrices,
• des capteurs OLED, en particulier dans des capteurs de gaz et de vapeur non isolés hermétiquement de l'extérieur,
• des diodes organiques,
• des cellules solaires organiques,
• des transistors organiques,
• des transistors à effet de champ organiques,
• des lasers organiques et
• des éléments d'abaissement de fréquence.

7. Utilisation selon la revendication 5 ou 6, dans laquelle la concentration de la molécule organique en tant qu'émetteur dans des composants luminescents optiques, en particulier dans des OLED, est comprise entre 5 % et 80 % en poids.

8. Composant optoélectronique, comportant une molécule organique selon l'une quelconque des revendications 1 à 4, en particulier configuré sous forme d'un composant choisi dans le groupe constitué par des diodes électroluminescentes organiques (OLED), une cellule électrochimique photoémettrice, un capteur OLED, en particulier des capteurs de gaz et de vapeur non isolés hermétiquement de l'extérieur, des diodes organiques, une cellule solaire organique, un transistor organique, un transistor à effet de champ organique, un laser organique et un élément d'abaissement de fréquence.

9. Composant optoélectronique selon la revendication 8, comportant

- un substrat,
- une anode et
- une cathode, l'anode et la cathode étant appliquées sur le substrat, et
- au moins une couche photoémettrice qui est disposée entre l'anode et la cathode et qui contient une molécule organique selon l'une quelconque des revendications 1 à 4.

10. Composant optoélectronique selon la revendication 8 ou 9, dans lequel la couche photoémettrice comporte au moins un matériau hôte, où l'état singulet excité ($S_1$) et/ou l'état triplet excité ($T_1$) dudit au moins un matériau hôte est plus élevé que l'état singulet excité ($S_1$) et/ou que l'état triplet excité ($T_1$) de la molécule organique selon l'une quelconque des revendications 1 à 4.

11. Composant optoélectronique selon l'une quelconque des revendications 8 à 10, comportant au moins un matériau hôte consistant en un matériau selon l'une quelconque des revendications 1 à 4.

12. Composant optoélectronique selon l'une quelconque des revendications 8 à 11, dans lequel la couche photoémettrice comporte des matériaux fluorescents ou phosphorescents et dans lequel pour ce qui est des matériaux de la couche photoémettrice il s'agit de molécules organiques selon l'une quelconque des revendications 1 à 4.

13. Composant optoélectronique selon la revendication 12, dans lequel une molécule organique selon l'une quelconque des revendications 1 à 4 forme conjointement avec un matériau fonctionnel un exciplexe.

14. Composant optoélectronique selon l'une quelconque des revendications 8 à 13, dans lequel une molécule organique selon l'une quelconque des revendications 1 à 4 est utilisée en tant que couche de transport de charge.

15. Procédé pour la production d'un composant optoélectronique, dans lequel on utilise une molécule organique selon l'une quelconque des revendications 1 à 4, en effectuant la mise en oeuvre de la molécule organique en particulier par un procédé de dépôt sous vide en phase vapeur ou à partir d'une solution.

Figur 1

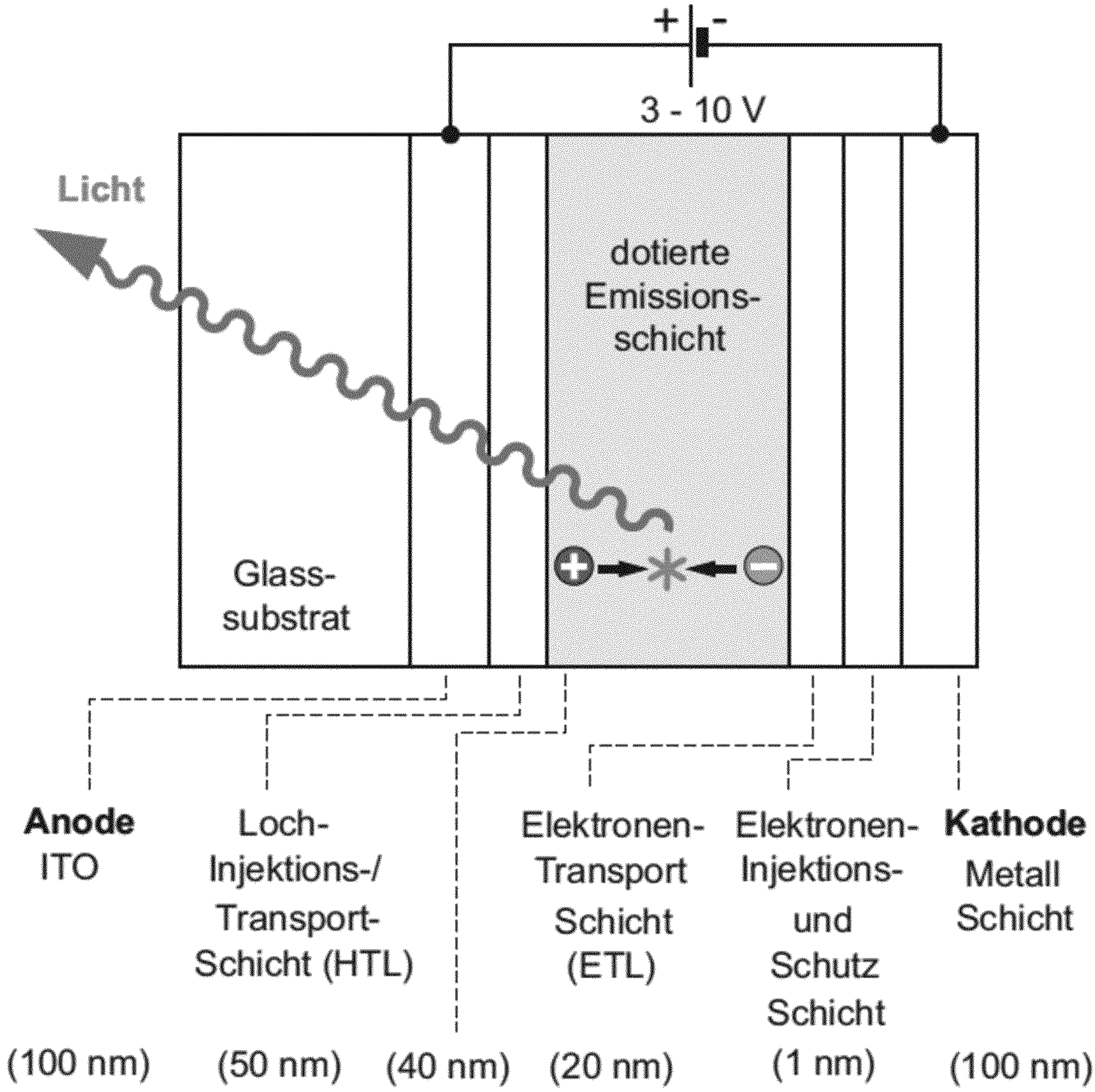
+
-
3 - 10 V
Licht
dotierte Emissions-schicht
Glass-substrat
Anode ITO (100 nm)
Loch-Injektions-/Transport-Schicht (HTL) (50 nm)
(40 nm)
Elektronen-Transport Schicht (ETL) (20 nm)
Elektronen-Injektions- und Schutz Schicht (1 nm)
Kathode Metall Schicht (100 nm)

Figur 2

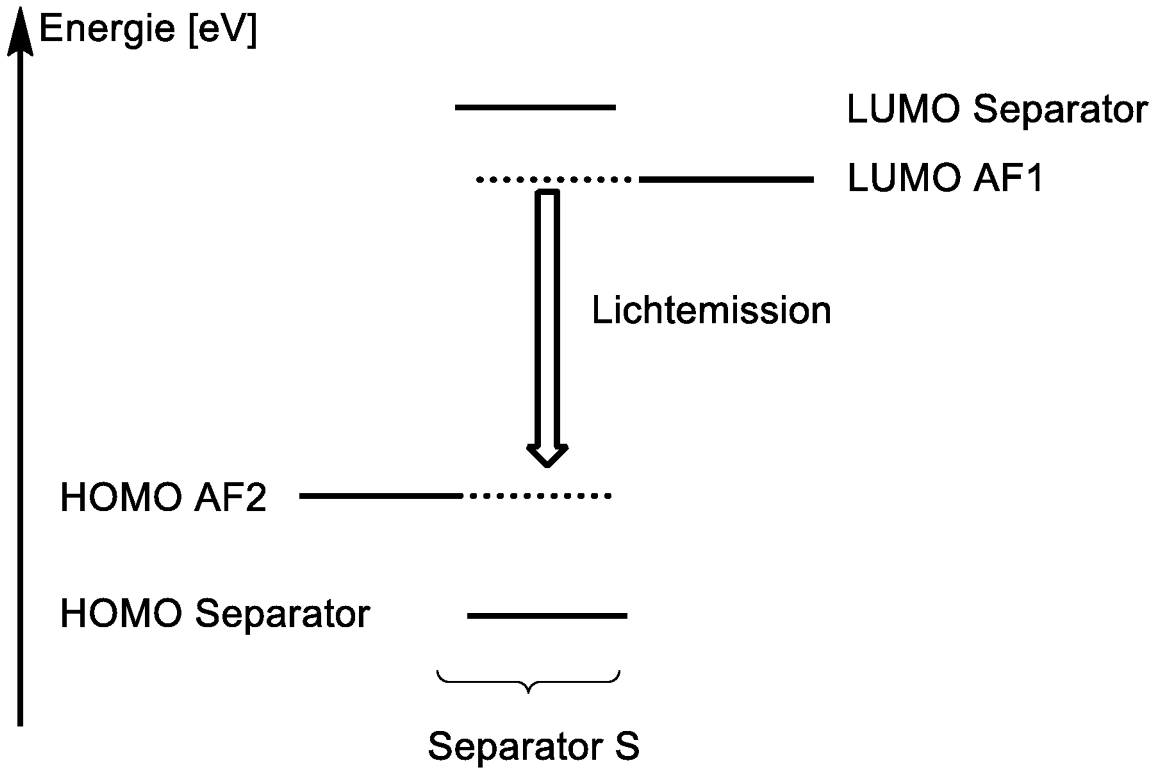
Energie [eV]
LUMO Separator
LUMO AF1
Lichtemission
HOMO AF2
HOMO Separator
Separator S

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2141152 A1
- EP 2461390 A1


### In der Beschreibung aufgeführte Nicht-Patentliteratur


- **H. YERSIN.** Top. Curr. Chem. 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **H. YERSIN.** *Top. Curr. Chem.,* 2004, vol. 241, 1
- **M. A. BALDO ; D. F. O'BRIEN ; M. E. THOMPSON ; S. R. FORREST.** *Phys. Rev. B,* 1999, vol. 60, 14422
- **SUGIMORI et al.** *Chemistry Letters,* 2000, vol. 10, 1200-1201
- **BECKE, A. D.** *Phys. Rev. A,* 1988, vol. 38, 3098-3100
- **PERDEW, J. P.** *Phys. Rev. B,* 1986, vol. 33, 8822-8827
- **WEIGEND, F. ; AHLRICHS, R.** *Phys. Chem. Chem. Phys.,* 2005, vol. 7, 3297-3305
- **RAPPOPORT, D. ; FURCHE, F. J.** *Chem. Phys.,* 2010, vol. 133, 134105, , 1-134105, 11
- **HÄSER, M. ; AHLRICHS, R. J.** *Comput. Chem.,* 1989, vol. 10, 104-111
- **WEIGEND, F. ; HÄSER, M.** *Theor. Chem. Acc.,* 1997, vol. 97, 331-340
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **RAPPOPORT, D. ; FURCHE, F.** *J. Chem. Phys.,* 2010, vol. 133, 134105, , 1-134105, 11